# EUROPEAN PATENT APPLICATION

(11) **EP 2 281 874 A1**
(43) Date of publication of application: **09.02.2011**
(21) Application number: 09166951.5
(22) Date of filing: 31.07.2009
(51) Int. Cl.: C12N 5/07, A61K 48/00, A61P 5/48

(54) **Production of ß-Cells**

(71) Applicant: Max-Planck-Gesellschaft zur Förderung der Wissenschaften e.V., 80539 München (DE)
(72) Inventor: Mansouri, Ahmed, 37085 Göttingen (DE); Collombat, Patrick, 06100 Nice (FR)
(74) Representative: Lahrtz, Fritz

(57) **Abstract**

The present invention relates to *in vitro* and in vivo methods for the generation of pancreatic β-cells, comprising the step of providing at least one pancreatic α-cell or at least one precursor cell with Pax-4 or a nucleic acid encoding Pax-4. Furthermore, the invention relates to a screening method for the screening of a pancreatic-β-cell phenotype-inducing compound, comprising the step of contacting at least one pancreatic α-cell or at least one precursor cell with a given compound, and testing whether said compound is capable of inducing a pancreatic-β-cell phenotype.

SEQUENCE LISTING

<110>  Max-Planck-Gesellschaft zur Förderung der Wissenschaften e.V.

<120>  Production of beta-cells

<130>  M66623EP

<160>  4

<170>  PatentIn version 3.5

<210>  1
<211>  28
<212>  DNA
<213>  Artificial Sequence

<220>
<223>  Primer pNgn3 Fw

<400>  1
caccaagctt tgtgtggaag gaaatgtc                                          28


<210>  2
<211>  24
<212>  DNA
<213>  Artificial Sequence

<220>
<223>  Primer pNgn3 Rv

<400>  2
cgcgcgcccc tcatccaccc tttg                                              24


<210>  3
<211>  21
<212>  DNA
<213>  Artificial Sequence

<220>
<223>  eGFP specific primer Fw

<400>  3
gacgtaaacg gccacaagtt c                                                 21


<210>  4
<211>  21
<212>  DNA
<213>  Artificial Sequence

<220>
<223>  eGFP specific primer Rev

<400>  4
gtcgccctcg aacttcacct c                                                 21

## Description

The present invention relates to *in vitro* and *in vivo* methods for the production of pancreatic β-cells as well as to methods for the screening of compounds inducing a pancreatic β-cell phenotype.

### BACKGOUND OF THE INVENTION

In humans, plasma glucose levels are regulated by the action of insulin, a peptide hormone that is produced and secreted by the pancreatic β-cells in response to nutrition. Diabetes mellitus, which comprises a heterogeneous group of hyperglycaemic disorders, results from inadequate mass and function of pancreatic β-cells. Worldwide prevalence figures give an estimate of 151 million cases in 2000 and an extrapolated number of 221 million in 2010 (Simmet, 2001, for the list of references see the example section). There are two forms of diabetes, namely type I diabetes linked to a selective autoimmune destruction of pancreatic β-cells and type II diabetes which is a severe disease of intermediary metabolism usually caused by β-cell dysfunction and/or resistance to the biological actions of insulin on its main target tissues, i.e. liver, muscle and fat. Current therapy for type I diabetes usually requires the treatment with insulin while the current therapy for type II diabetes may additionally include the modification of lifestyle such as diet and exercise and the use pharmacological agents that stimulate insulin secretion. However, to date, there is no cure available for the treatment of diabetes.

The endocrine pancreas is organized in islets of Langerhans comprising five original cell subtypes, α-, β-, δ-, ε-, and PP-cells, secreting glucagon, insulin, somatostatin, ghrelin, and pancreatic polypeptide (PP), respectively. The identification and characterization of the genetic determinants underlying endocrine pancreas morphogenesis and regeneration may potentially aid designing cell replacement therapies to treat type 1 and 2 diabetes. In this context, and as also described in detail in the example by reference to numerous scientific publications, a number of studies have demonstrated that, during development, the cooperation of several transcription factors successively specifies progenitor cells towards the pancreatic-, endocrine- and ultimately islet-cell fates, indicating that the formation of the different cell types of the pancreas is due to the activities of a complex network of transcription factors. Furthermore, it has been shown in the art that Pax-4 is able to induce proliferation of pancreatic β-cells (WO 06/015853). However, this document is silent about the transdifferentiation of α-cells or precusur cells into β-cells.

There is an urgent need for providing further means and methods for the treatment of diabetes.

### SUMMARY OF THE INVENTION

The present invention relates to an *in vitro* method for the generation of pancreatic β-cells, comprising the step of providing at least one pancreatic α-cell or at least one precursor cell with a pancreatic β-cell phenotype-inducing compound.

The present invention relates to an *in vitro* method for the generation of pancreatic β-cells, comprising the step of providing at least one pancreatic α-cell or at least one precursor cell with Pax-4 or a nucleic acid encoding Pax-4.

In a preferred embodiment, said method includes providing at least one pancreatic α-cell or at least one precursor cell as the starting material.

In a preferred embodiment, the precursor cell is a pancreatic precursor cell, a small intestine precursor cell, a liver precursor cell or a precursor cell contained within the duct population.

In a preferred embodiment, said nucleic acid is a DNA.

In a preferred embodiment, the DNA is comprised in a vector.

In a preferred embodiment, said nucleic acid is an mRNA.

In a preferred embodiment, said method does not include providing other proteins involved in cellular development or nucleic acids encoding proteins involved in cellular development.

In a preferred embodiment, said method does not include providing other proteins apart from marker proteins or other genes apart from genes encoding marker proteins.

The present invention further relates to a pancreatic β-cell phenotype inducing compound for use in a method for the generation of pancreatic β-cells *in vivo,* wherein said method includes providing at least one pancreatic α-cell or at least one precursor cell with a pancreatic β-cell phenotype-inducing compound *in vivo.*

The present invention further relates to Pax-4 or a nucleic acid encoding Pax-4 for use in a method for the generation of pancreatic β-cells *in vivo,* wherein said method includes providing at least one pancreatic α-cell or at least one precursor cell with Pax-4 or a nucleic acid encoding Pax-4 *in vivo.*

The invention further relates to a method for the generation of pancreatic β-cells *in vivo,* wherein said method includes providing at least one pancreatic α-cell or at least one precursor cell with Pax-4 or a nucleic acid encoding Pax-4 *in vivo.*

In a preferred embodiment, said nucleic acid is a DNA.

In a preferred embodiment, the DNA is comprised in a vector.

In a preferred embodiment, said nucleic acid is an mRNA.

In a preferred embodiment, said method does not include providing other proteins involved in cellular development or nucleic acids encoding proteins involved in cellular development.

In a preferred embodiment, said method does not include providing other proteins apart from marker proteins or other genes apart from genes encoding marker proteins.

In a preferred embodiment, said method is for the treatment of a pancreatic disease.

In a preferred embodiment, said pancreatic disease is diabetes.

The invention further relates to a method for the screening of a pancreatic-β-cell phenotype-inducing compound, comprising the step of
a) contacting at least one pancreatic α-cell or at least one precursor cell with a given compound, and
b) testing whether said compound is capable of inducing a pancreatic-β-cell phenotype.

In a preferred embodiment, said testing includes determining whether after the contacting the cell is capable of producing insulin.

In a preferred embodiment, said testing includes determining whether after the contacting the expression of Pax-4 is increased in the cell.

In a preferred embodiment, said method is performed *in vitro.*

### DETAILED DESCRIPTION OF THE INVENTION

In the context of the present invention, it has been surprisingly found that induction of the expression of Pax-4 in pancreatic adult α-cells or pancreatic precursor cells results in the generation of pancreatic β-cells *in vivo* (cf. the example). This enables *in vitro* and *in vivo* methods for the generation of pancreatic β-cells as well as screening methods for pancreatic β-cell phenotype inducing compounds.

"Pancreatic β-cells" or "β-cells" are well known in the art (for the respective literature see the example) and are, in the context of the present invention, cells capable of producing insulin upon stimulation with glucose.

In the context of the present invention, a pancreatic β-cell phenotype-inducing compound is provided to α-cells or precursor cells. After said provision, the level of said compound is increased in cell, with the consequence that the cell differentiates to a pancreatic β-cell phenotype. In the context of the present invention, the term "pancreatic β-cell phenotype" means that the cell is capable of producing insulin upon stimulation with glucose.

Method for determining whether a cell has a pancreatic β-cell phenotype are known in the art and include incubating the cell with glucose and testing whether insulin expression in the cell is increased or started (Nolan, 2009).Other methods include the testing whether β-cell specific transcription factors are expressed, the detection of β-cell specific gene products with the help of RNA quantitative PCR, the transplantation of a candidate cell in diabetic mice, and subsequent testing of the physiologic response following said transplantation as well analysing the cells with electron microscopy. These methods are all known in the art.

In principle, the pancreatic β-cell phenotype-inducing compound may be a compound of any type. This includes a protein, a nucleic acid, but also other compounds like small organic molecules, especially in the context of the screening claims of the invention (see below.)

In a preferred embodiment of the invention, Pax-4 or a nucleic acid encoding Pax-4 are provided to the cell.

The functional, wild-type Pax-4 to be particularly used in context of this invention is the wildtype Pax-4 of mouse, rat or human. Most particularly, the functional, wild-type Pax-4 to be employed is human Pax-4. The corresponding sequences (*i*.*e*., nucleotide sequences and amino acid sequences) are known in the art (see below and the example section).

In accordance with this invention, also Pax-4 molecules may be employed which are highly homologous to the functional, wild-type Pax-4 molecules known in the art and disclosed herein. Yet, these molecules have to be functional in the sense that these molecules do stimulate the generation of β-cells as documented herein. Accordingly, the invention also provides for a "read-out System" whether a given Pax-4 molecule is "functional", *i*.*e*., capable of generating β-cells starting from α-cells or precursor cells.

Functional, wild-type Pax-4 molecules are known in the art, and comprise, but are not limited to the sequences disclosed in U.S. Patent 6,071,697. Further "functional, wild-type Pax4" molecules are encoded by nucleic acid molecules as provided in the GeneBank under accession numbers: (rat Pax-4) NM_031799 (mousePax-4) NM_011038 and (humanPax-4): AF043978 orNM_006193.

In a preferred embodiment, these Pax-4 molecules may have a sequence identity of at least 40%, particularly at least 50%, more particularly at least 60%, even more particularly at least 70%, particularly at least 80%, more particularly at least 90%, even more particularly at least 95%», 97% or 98% and most particularly at least 99% identity with a nucleic acid sequence as described above encoding a wild-type Pax-4.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same *(e*.*g*., 70-95% identity, more particularly at least 95%, 97%, 98% or 99% identity), when compared and aligned for maximum correspondence over a window of comparison, or over a designated region as measured using a sequence comparison algorithm as known in the art, or by manual alignment and visual inspection. Sequences having, for example, 95% or greater sequence identity are considered to be substantially identical. Such a definition also applies to the complement of a test sequence. Those having skill in the art will know how to determine percent identity between/among sequences using, for example, algorithms such as those based on CLUSTALW Computer program (Thompson, 1994; or FASTDB (Brutlag, 1990, as known in the art)).

In case that a nucleic acid encoding Pax-4 is used in the context of the present invention, this nucleic acid is preferably a DNA, which is preferably comprised in a vector, especially an expression vector. However, it is also envisaged that the Pax-4 encoding nucleic acid is provided per se to the cell or cells, and is then integrated into the cellular DNA e.g. by homologous recombination.

Consequently, "recombinant vectors" or "expression vectors" or "expression constructs" form important further embodiments of the present invention. The terms "expression vector", "recombinant vector" or "expression construct" are used interchangeably in the context of the present invention and mean any type of genetic construct containing a nucleic acid coding for a gene product in which part or all of the nucleic acid encoding sequence is capable of being transcribed, and later on translated, either entirely due to information provided on the vector or construct or also partially due to information provided by the host cell.

Particularly useful vectors are contemplated to be those vectors in which the coding portion of the DNA segment, whether encoding a full length protein or smaller peptide, is positioned under the transcriptional control of a promoter. A "promoter" refers to a DNA sequence recognized by the synthetic machinery of the cell, or introduced synthetic machinery, required to initiate the specific transcription of a gene. The phrases "operatively positioned", "under control" or "under transcriptional control" means that the promoter is in the correct location and orientation in relation to the nucleic acid to control RNA polymerase initiation and expression of the gene. The promoter may be in the form of the promoter that is naturally associated with a particular gene, as may be obtained by isolating the 5' non-coding sequences located upstream of the coding segment or exon, for example, using recombinant cloning and/or polymerase chain reaction (PCR™) technology.

In other embodiments, it is contemplated that certain advantages will be gained by positioning the coding DNA segment under the control of a recombinant, or heterologous, promoter. As used herein, a recombinant or heterologous promoter is intended to refer to a promoter that is not normally associated with a particular gene in its natural environment. Such promoters may include promoters normally associated with other genes, and/or promoters isolated from any other bacterial, viral, eukaryotic, or mammalian cells.

Naturally, it will be important to employ a promoter that effectively directs the expression of the DNA segment in the cell type chosen for expression. The use of promoter and cell type combinations for protein expression is generally known to those of skill in the art of molecular biology, for example, see Sambrook (2001, which can be used as a reference for all molecular biology methods described herein). The promoters employed may be constitutive, or inducible, and can be used under the appropriate conditions to direct high level expression of the introduced DNA segment, such as is advantageous in the large-scale production of recombinant proteins or peptides.

The promoter is required to express the transforming genetic construct to a degree sufficient to effect transformation of a target cell type amongst a population of different cell types such that the transformed target cell results in the generation of a stable human regulated secretory cell. Promoters can be classified into two groups, ubiquitous and tissue- or cell-specific. Ubiquitous promoters activate transcription in all or most tissues and cell types. Examples of ubiquitous promoters are cellular promoters like the histone promoters, promoters for many metabolic enzyme genes such as hexokinase I and glyceraldehyde-3-phosphate dehydrogenase, and many viral promoters such as the cytomegalovirus promoter (CMVp) and the Rous sarcoma virus promoter (RSVp).

Tissue- or cell-specific promoters activate transcription in a restricted set of tissues or cell types or, in some cases, only in a single cell type of a particular tissue.

Alternatively, the nucleic acid encoding Pax-4 may be an mRNA.

As it can be taken from the examples, the inventors of the present invention have been able to show that the transfection of Pax-4 encoding nucleic acids is sufficient for the generation of a pancreatic β-cell phenotype, i.e. for the differentiation of pancreatic α-cells or precursor cells into pancreatic β-cells. Consequently, in a preferred embodiment of the invention, Pax-4 or a nucleic acid encoding Pax-4 are the only functional agents provided to the cells.

In this context, the term "no other functional agent" means that no other agent is added which is involved in the differentiation into a pancreatic β-cell phenotype.

In a further preferred embodiment, this means that said method does not include providing other proteins involved in cellular development or nucleic acids encoding proteins involved in cellular development or that said method does not include providing other proteins apart from marker proteins or protein conferring resistance against antibiotics or other genes nucleic acid sequences apart from genes encoding marker proteins or proteins conferring resistance against antibiotics or regulatory sequences. It will be appreciated by the person skilled in the art that when a nucleic acid encoding Pax-4 is transfected, other nucleic acids may also be transfected which includes marker genes, antibiotic resistance genes or regulatory sequences. These nucleic acids, however, do not have any impact on the differentiation into the pancreatic β-cell phenotype.

In the context of the methods of the invention, compounds and in particular Pax-4 or nucleic acid encoding Pax-4 are provided to at least one pancreatic α-cell or at least one precursor cell.

Methods for providing a protein, and especially a transcription factor like Pax-4, to cells are well known in the art and include incubation of the respective cell with a liposome-containing protein, electroporation (Amaxa, Lonza, Nucleofector) as well as delivery through the HIV TAT system (Zhao, 2007).

Methods for providing the respective cell with a nucleic acid encoding Pax-4 are also well known in the art and include preferably transfection methods and viral infections.

In order to effect expression, the nucleic acid must be delivered into a cell. As described below, a possible mechanism for delivery is *via* viral infection, where the nucleic acid is encapsi-dated in an infectious viral particle. However, several non-viral methods for the transfer of the nucleic acid into cultured mammalian cells (transfection methods) also are contemplated by the present invention. In one embodiment of the present invention, the nucleic acid may consist only of naked recombinant DNA or plasmids. Transfer of the vector may be performed by any of the methods mentioned which physically or chemically permeabilize the cell membrane.

In a further embodiment of the invention, the nucleic acid may be entrapped in a liposome. Liposomes are vesicular structures characterized by a phospholipid bilayer membrane and an inner aqueous medium. Multilamellar liposomes have multiple lipid layers separated by aqueous medium. They form spontaneously when phospholipids are suspended in an excess of aqueous solution.

In certain embodiments of the present invention, the nucleic acid is introduced into the cell *via* electroporation. Electroporation involves the exposure of a Suspension of cells and DNA to a high-voltage electric discharge. Transfection of eukaryotic cells using electroporation has been quite successful in the art (see e.g. Sambrook (2001)).

In other embodiments of the present invention, the nucleic acid is introduced to the cells using calcium phosphate precipitation, as generally in the art. Other transfection methods include the use of DEAE-dextran followed by polyethylene glycol or particle bombardment.

The vector expressing a nucleic acid encoding Pax-4 as defined above to be used in an *in vi-trio* or an *in vivo* method of the invention in accordance with this invention may also be a viral vector. In this context, the vector may be selected from the group consisting of an adenoviral vector, an adeno-associated virus vector, a vaccinia virus vector, a herpes virus vector, a retroviral vector or an lentiviral vector , but also other viral vectors known in the art may be used. Such vectors are known in the art (Gene Transfer, Delivery and Expression of DNA and RNA, A Laboratory manual, Edit by T. Friedmann, J. Rossi, Cold Spring Harbor Laboratory Press, New York 2006), Hitt (2006), Salmon (2006)). Viral vector are especially preferred in the *in vivo* methods of the present invention.

According to the invention, the compound is provided to at least one pancreatic α-cell or at least one precursor cell. Consequently, in a preferred embodiment, first at least one pancreatic α-cell or at least one precursor cell are provided as the starting material. In a further preferred embodiment, the starting material is at least one isolated α-cell or at least one isolated precursor cell.

"α-cells" or "pancreatic α-cells" are known in the art (see also the example for literature) and are capable of expressing glucagon as a consequence of decreased blood sugar levels. Consequently, in the context of the present invention, an α-cell is preferably a cell capable of expressing glucagon as a consequence of decreased blood sugar levels.

Alternatively, also other adult cells, especially liver cells, duct cells or intestinal cells can be used as the starting material of the method of the present invention.

In the context of the present invention, the term "isolated" means that the respective cell has been at least partially purified.

Methods for providing at least one α-cell are known in the art and include isolating pancreatic tissue and isolating the cell e.g. with the help of FACS (cell sorter) as known in the art or providing a α-cell-line (Powers, 1990).

Methods for providing precursor cells are also known in the art and include either providing tissue containing said precursor cells and isolating the cells by methods known in the art, e.g. with the help of cell surface specific antibodies and using a FACS (cell sorter) or cultivation of the cells under specific conditions allowing the growth of precursor cells. Furthermore, also suitable cell lines are known in the art (Lieber (1975)).

Every precursor cell being capable of differentiating into pancreatic β-cells can be used as the starting cell of the method of the invention. Especially, this includes all precursor cells derived from the endoderm. In a preferred embodiment, said precursor cell is selected from the group consisting of a pancreatic precursor cell, a small intestine precursor cell, a liver precursor cell, a precursor cell derived from the duct population, and a pancreatic stem cell.

After the cells have been provided, said cell or cells may be present in any in vitro cell culture system useful for the context of the present invention. This includes standard cell culture systems like tissue culture dishes as well as 6-well, 24-well or 96-well plates. Culture conditions will depend on the respective cell. The person skilled in the art will know how to cultivate the cells.

In the context of the present invention, it has been shown that an increase of functional Pax-4 in a pancreatic α-cell or a precursor cell results in the formation of pancreatic β-cells. In the examples of the present invention, this increase in functional Pax-4 has been obtained by providing a nucleic acid encoding Pax-4 *in vivo.* However, as outlined above, it is equally feasible to provide Pax-4 protein itself. Furthermore, it is feasible to provide any other compound which is able to induce a pancreatic β-cell phenotype. Consequently, the finding of the inventors of the present invention that it is possible to generate pancreatic β-cells out of pancreatic α-cells or precursor cells enables also a screening method for pancreatic β-cell phenotype inducing compounds.

Consequently, in another aspect of the present invention, the invention relates to a method for the screening of a pancreatic β-cell phenotype inducing compound comprising the steps of
(a) contacting at least one pancreatic α-cell or at least one precursor cell with a given compound and
(b) testing whether said compound is capable of inducing a pancreatic β-cell phenotype.

In principle, with the method of the invention, it is possible to screen for every compound being capable of inducing a β-cell phenotype.

Preferably, said compound is selected from the group consisting of synthetic or naturally occurring chemical compounds or organic synthetic drugs, more preferably small molecule organic drugs or natural small molecule compounds. Preferably, said compound is identified starting from a library containing such compounds. Then, in the course of the present invention, such a library is screened.

Such small molecules are preferably not proteins or nucleic acids. Preferably, small molecules exhibit a molecular weight of less than 1000 Da, more preferred less than 750 Da, most preferred less than 500 Da.

A "library" according to the present invention relates to a (mostly large) collection of (numerous) different chemical entities that are provided in a sorted manner that enables both a fast functional analysis (screening) of the different individual entities, and at the same time provide for a rapid identification of the individual entities that form the library. Examples are collections of tubes or wells or spots on surfaces that contain chemical compounds that can be added into reactions with one or more defined potentially interacting partners in a high-throughput fashion. After the identification of a desired "positive" interaction of both partners, the respective compound can be rapidly identified due to the library construction. Libraries of synthetic and natural origins can either be purchased or designed by the skilled artisan.

Examples of the construction of libraries are provided in, for example, Breinbauer R, (2002), wherein natural products are described that are biologically validated starting points for the design of combinatorial libraries, as they have a proven record of biological relevance. This special role of natural products in medicinal chemistry and chemical biology can be interpreted in the light of new insights about the domain architecture of proteins gained by structural biology and bioinformatics. In order to fulfill the specific requirements of the individual binding pocket within a domain family it may be necessary to optimise the natural product structure by chemical variation. Solid-phase chemistry is said to become an efficient tool for this optimisation process, and recent advances in this field are highlighted in this review article. The current drug discovery processes in many pharmaceutical companies require large and growing collections of high quality lead structures for use in high throughput screening assays. Collections of small molecules with diverse structures and "drug-like" properties have, in the past, been acquired by several means: by archive of previous internal lead optimisation efforts, by purchase from compound vendors, and by union of separate collections following company mergers. Although high throughput/combinatorial chemistry is described as being an important component in the process of new lead generation, the selection of library designs for synthesis and the subsequent design of library members has evolved to a new level of challenge and importance. The potential benefits of screening multiple small molecule compound library designs against multiple biological targets offers substantial opportunity to discover new lead structures.

The compound to be screened is added to at least one α-cell or at least on precursor cell as described above for the method of the invention for the generation of β-cells. Furthermore, also the embodiments described above with respect to the providing of α-cells or precursor cells including the respective cell lines, especially with respect to their isolation, also apply to the screening method of the invention.

In the context of the screening claims of the present invention, it is particularly preferred that cells of a given suitable cell line, e.g. an α-cell line or a duct cell line, are cultivated and contacted with the compound. A suitable readout system could then be whether the cells are then capable of producing insulin. Another readout system could be to transfect the cells with a reporter gene under the control of a β-cell specific promoter, e.g. the insulin promoter, and testing whether the compounds are capable of inducing the expression of the reporter gene.

In the context of the screening method of the invention, it is tested whether a given compound is able to induce a pancreatic β-cell phenotype. In a preferred embodiment, said testing includes determining whether after the contacting the cells are capable of producing insulin, in particular after incubation of the cells with glucose. This readout of the screening of the invention is especially suitable because the capacity of producing insulin is the major feature of pancreatic β-cells.

However, since it has been shown that Pax-4 is able to induce a pancreatic β-cell phenotype if more functional Pax-4 is present in the cell (see above), said testing includes in a preferred embodiment also whether after the contacting the expression of Pax-4 is increased in the cell. In a preferred embodiment, the level of Pax-4 is compared to the level of Pax-4 in a cell not contacted with the compound.

In an especially preferred embodiment, said testing includes both the capacity of producing insulin as well determining the expression of Pax-4.

In a further preferred embodiment of this aspect of the invention, said screening method is performed *in vitro,* preferably in the context of a medium- or high-throughput system. However, it is equally envisaged to use animal models for the testing of compounds. In this context, animal models of diabetes as known in the art are especially suitable and include NOD mice , streptozotocin treated mice, as well as mice treated with diphtheria toxin.

As shown in the examples of the present invention, with the help of Pax-4 or nucleic acid encoding Pax-4, it is also possible to generate pancreatic β-cells *in vivo.* Consequently, the present invention also relates to the *in vivo* application of a pancreatic β-cell phenotype-inducing compound, especially Pax-4 or a nucleic acid encoding Pax-4. This compound may be preferably contained in a pharmaceutical composition.

In general, the pharmaceutical compositions of the present invention comprise a therapeutically effective amount of a therapeutic, and a pharmaceutically acceptable carrier. In a specific embodiment, the term "pharmaceutically acceptable" means approved by a regulatory agency of the Federal or a state government or listed in the U.S. Pharmacopeia or other generally recognized pharmacopeias for use in animals, and more particularly, in humans. The term "carrier" refers to a diluent, adjuvant, excipient, or vehicle with which the therapeutic is administered. Such pharmaceutical carriers can be sterile liquids, such as water and oils, including those of petroleum, animal, vegetable or synthetic origin, including but not limited to peanut oil, soybean oil, mineral oil, sesame oil and the like. Water is a preferred carrier when the pharmaceutical composition is administered orally. Saline and aqueous dextrose are preferred carriers when the pharmaceutical composition is administered intravenously. Saline solutions and aqueous dextrose and glycerol solutions are preferably employed as liquid carriers for injectable solutions. Suitable pharmaceutical excipients include starch, glucose, lactose, sucrose, gelatin, malt, rice, flour, chalk, silica gel, sodium stearate, glycerol monostearate, talc, sodium chloride, dried skim milk, glycerol, propylene, glycol, water, ethanol and the like. The composition, if desired, can also contain minor amounts of wetting or emulsifying agents, or pH buffering agents. These compositions can take the form of solutions, suspensions, emulsions, tablets, pills, capsules, powders, sustained-release formulations and the like. The composition can be formulated as a suppository, with traditional binders and carriers such as triglycerides. Oral formulation can include standard carriers such as pharmaceutical grades of mannitol, lactose, starch, magnesium stearate, sodium saccharine, cellulose, magnesium carbonate, etc.. Such compositions will contain a therapeutically effective amount of the therapeutic, preferably in purified form, together with a suitable amount of carrier so as to provide the form for proper administration to the patient. The formulation should suit the mode of administration.

In a preferred embodiment, the composition is formulated, in accordance with routine procedures, as a pharmaceutical composition adapted for intravenous administration to human beings. Typically, compositions for intravenous administration are solutions in sterile isotonic aqueous buffer. Where necessary, the composition may also include a solubilizing agent and a local anaesthetic such as lidocaine to ease pain at the site of the injection. Generally, the ingredients are supplied either separately or mixed together in unit dosage form, for example, as a dry lyophilized powder or water-free concentrate in a hermetically sealed container such as an ampoule or sachette indicating the quantity of active agent. Where the composition is to be administered by infusion, it can be dispensed with an infusion bottle containing sterile pharmaceutical grade water or saline. Where the composition is administered by injection, an ampoule of sterile water or saline for injection can be provided so that the ingredients may be mixed prior to administration.

The therapeutics of the invention can be formulated as neutral or salt forms. Pharmaceutically acceptable salts include those formed with free carboxyl groups such as those derived from hydrochloric, phosphoric, acetic, oxalic, tartaric acids, etc., those formed with free amine groups such as those derived from isopropylamine, triethylamine, 2-ethylamino ethanol, histidine, procaine, etc., and those derived from sodium, potassium, ammonium, calcium, and ferric hydroxides, etc..

The amount of the therapeutic of the invention which will be effective in the treatment of a particular disorder or condition will depend on the nature of the disorder or condition, and can be determined by standard clinical techniques. In addition, in vitro assays may optionally be employed to help identify optimal dosage ranges. The precise dose to be employed in the formulation will also depend on the route of administration, and the seriousness of the disease or disorder, and should be decided according to the judgment of the practitioner and each patient's circumstances. However, suitable dosage ranges for intravenous administration are generally about 20-500 micrograms of active compound per kilogram body weight. Suitable dosage ranges for intranasal administration are generally about 0.01 pg/kg body weight to 1 mg/kg body weight. Effective doses may be extrapolated from dose-response curves derived from in vitro or animal model test systems. In general, suppositories may contain active ingredient in the range of 0.5% to 10% by weight; oral formulations preferably contain 10% to 95% active ingredient.

Various delivery systems are known and can be used to administer a therapeutic of the invention, e.g., encapsulation in liposomes, microparticles, and microcapsules: use of recombinant cells capable of expressing the therapeutic, construction of a therapeutic nucleic acid as part of a retroviral or other vector, etc. Methods of introduction include but are not limited to intradermal, intramuscular, intraperitoneal, intravenous, subcutaneous, intranasal, epidural, and oral routes. The compounds may be administered by any convenient route, for example by infusion, by bolus injection, by absorption through epithelial or mucocutaneous linings (e.g., oral, rectal and intestinal mucosa, etc.), and may be administered together with other biologically active agents. Administration can be systemic or local.

In a specific embodiment, it may be desirable to administer the pharmaceutical compositions of the invention locally to the area in need of treatment. This may be achieved by, for example, and not by way of limitation, local infusion during surgery, topical application, e.g., in conjunction with a wound dressing after surgery, by injection, by means of a catheter, by means of a suppository, or by means of an implant, said implant being of a porous, nonporous, or gelatinous material, including membranes, such as sialastic membranes, or fibers. In one embodiment, administration can be by direct injection at the site (or former site) of a malignant tumor or neoplastic or pre-neoplastic tissue.

In another embodiment, the therapeutic can be delivered in a vesicle, in particular a liposome (Langer, (1990)).

In a further preferred embodiment of the aspect of the invention where Pax-4 or nucleic acid encoding Pax-4 is provided *in vivo,* said method is for the treatment of a pancreatic disease, preferably diabetes.

The invention is further illustrated by the following figures and examples which are intended to explain, but not to limit the scope of the present invention.

### FIGURE LEGENDS

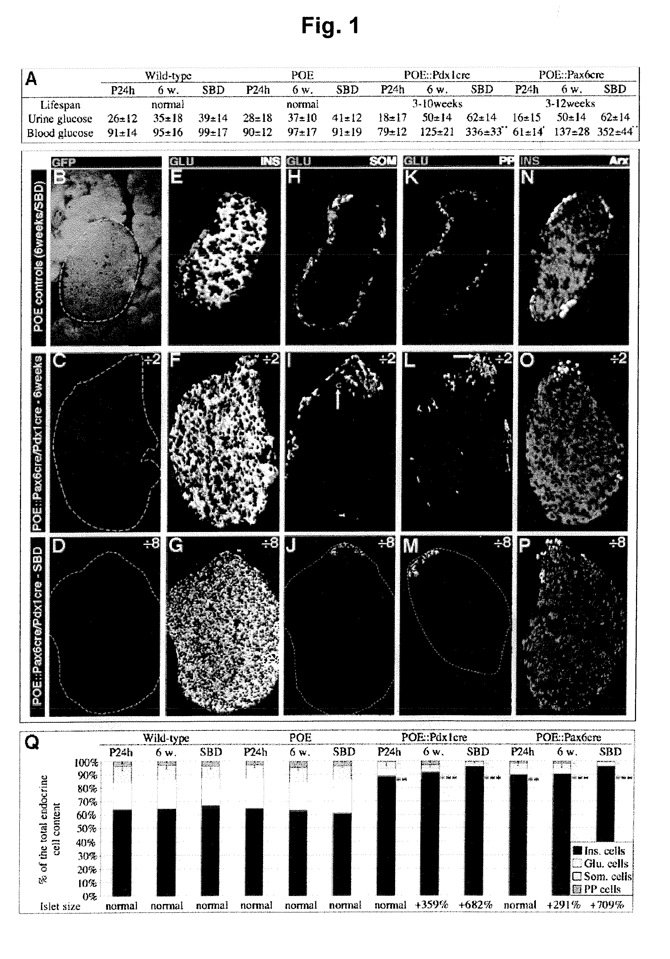

### Figure 1. The ectopic expression of Pax4 in Pdx1 or Pax6 expression domains promotes the genesis of oversized islets mainly containing insulin-expressing cells.

(A) Characterization of the lifespan and glycemia (in mg/dl) of POE::Pdxlcre and POE::Pax6cre mice 1 day and 6 weeks *postpartum*, as well as short before death (SBD). Note that initially, these animals are hypo- and subsequently hyperglycemic. (B-P) Immunohistochemical analysis of the islets of double-transgenic mice. A dramatic increase in islet size and insulin-expressing cell mass is evident 6 weeks *postpartum* (C, F, I, L, O) and is even more pronounced SBD (D, G, J, M, P) as compared to representative control islets (B, E, H, K, N). Concurrently, a loss of α- (E-M), δ- (H-J), PP- (K-M), and Arx-labeled (N-P) cells is evident, but, interestingly, few of these remain detectable at one pole of the islet, some co-expressing the glucagon and somatostatin (arrow in I) or PP (arrow in L) hormones. (Q) A quantification of the endocrine cell alterations ascertains these observations (also see Table 2). For the purpose of clarity, the magnification of double-transgenic islets is twice (C, F, I, L, O) or eight times (D, G, J, M, P) reduced compared to controls. (n>11, * P<0.05, ** P<0.01, *** P<0.001, all values expressed as means ± standard error of the mean).

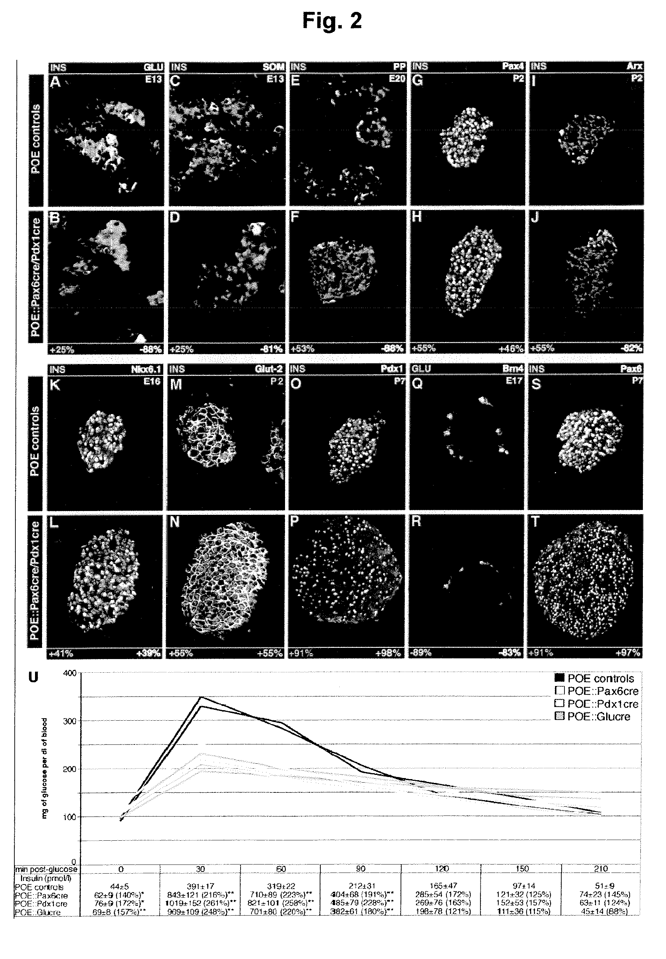

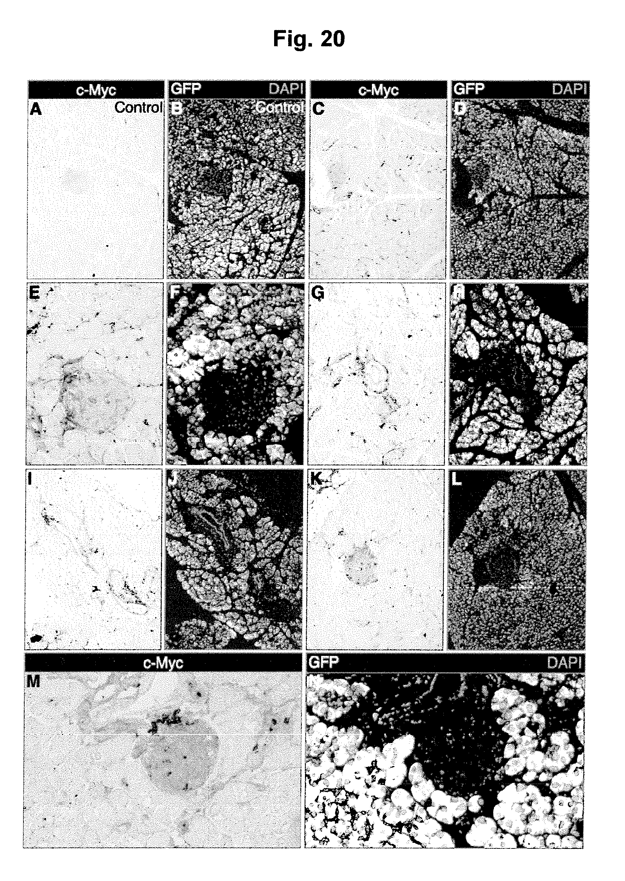

### Figure 2. Insulin-expressing cells detected in POE::Pdxlcre and POE::Pax6cre pancreata exhibit a β-cell identity.

(A-T) Characterization of POE::Pdxlcre and POE::Pax6cre hormone-expressing cells. Sections of double-transgenic pancreata (of the indicated ages) were stained using the mentioned antibodies, the number of labeled cells counted and reported to the count obtained in POE control animals. The values are expressed in percentage of change in labeled cell number compared to controls. All values are statistically significant with P values lower than or equal to 0.05. Note that the numbers of insulin-expressing cells are dramatically increased in double-transgenic animals at all examined stages (A-P, S-T) and this, as early as E13 (A-B), compared to controls. Concurrently, the mean contents of α- (A-B, G-J, Q-R), δ- (C-D), PP-(E-F) and Arx- (I-J) marked cells appear drastically reduced. A thorough analysis of endocrine cell-specific markers demonstrates that, all insulin-producing cells clearly express Pax4 (G-H), as well as the β-cell markers Nkx6.1 (K-L), Glut-2 (M-N) and Pdx1 (O-P). These are negative for the α-cell specific factor Brn-4 (Q-R), whereas, all endocrine cells express Pax6 (S-T). (U) An improved glucose tolerance and insulin release are highlighted in 3-week-old double-transgenic mice as compared to controls POE mice (n>3, ***P<0.001, **P<0.01, * P<0.05). Each picture is representative of at least 4 independent animals; note that, for the purpose of clarity, a POE::Pax6cre islet is displayed in H.

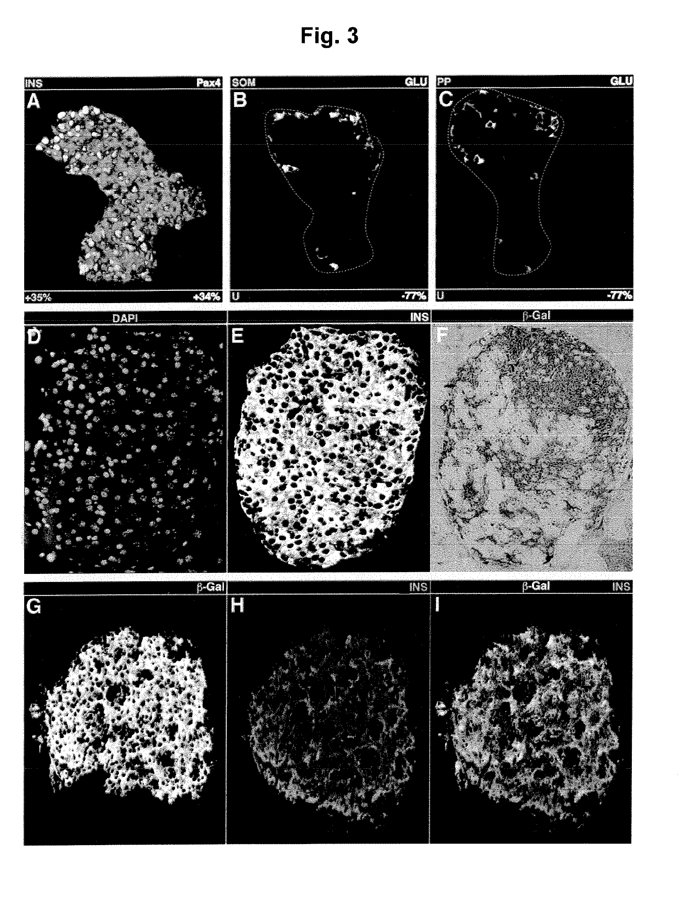

### Figure 3. Conversion of glucagon-expressing cells into insulin-producing cells upon Pax4 ectopic expression.

(A-C) Quantification of the endocrine cell content alterations upon ectopic expression of the *Pax4* gene in glucagon-producing cells in 1-week-old animals. A clear increase in the number of insulin-/Pax4-labeled cells at the expense of glucagon-expressing cells is highlighted (A), whereas the δ- and PP-cell contents are found unchanged (B-C). Note the accumulation of the remaining glucagon-marked cells at one pole of the islet. (D-F) The detection of insulin- and β-galactosidase-expressing cells on serial sections demonstrates that numerous insulin-labeled cells do express the β*-galactosidase* gene that normally marks glucagon-positive cells. (G-I) The same observation is made in 6-week old animals using co-immunofluorescence. All reported values are statistically significant (P<0.05, n=5). See Table 2 for a detailed analysis.

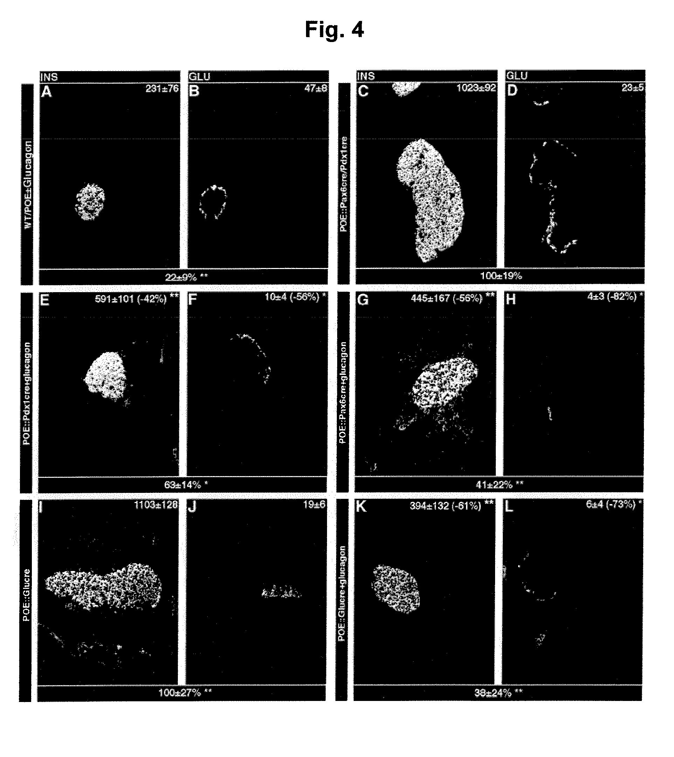

### Figure 4. Exogenous glucagon supplementation prevents islet overgrowth.

Six-week old pancreata of the indicated genotypes were sectioned and stained using anti-insulin (A, C, E, G, I, K) or glucagon (B, D, F, H, J, L) antibodies. The values at the top right corner of each photograph represent the number of hormone-producing cells per square centimeter of pancreas. The counts reported underneath each picture set represent variations in islet surface (estimated *in silico*) using POE::Pax6cre/Pdxlcre pancreata (C-D) as reference for photographs A-H and POE::Glucre pancreata (I-J) as reference for photographs I-L. Note the overall diminution in islet size and glucagon⁺ cell content, as well as the drastic decrease in insulin-expressing cell number (E-H compared to C-D and K-L compared to I-J) in animals supplemented with glucagon for 3 weeks compared to untreated ones (*P<0.05, **P<0.01, n=3).

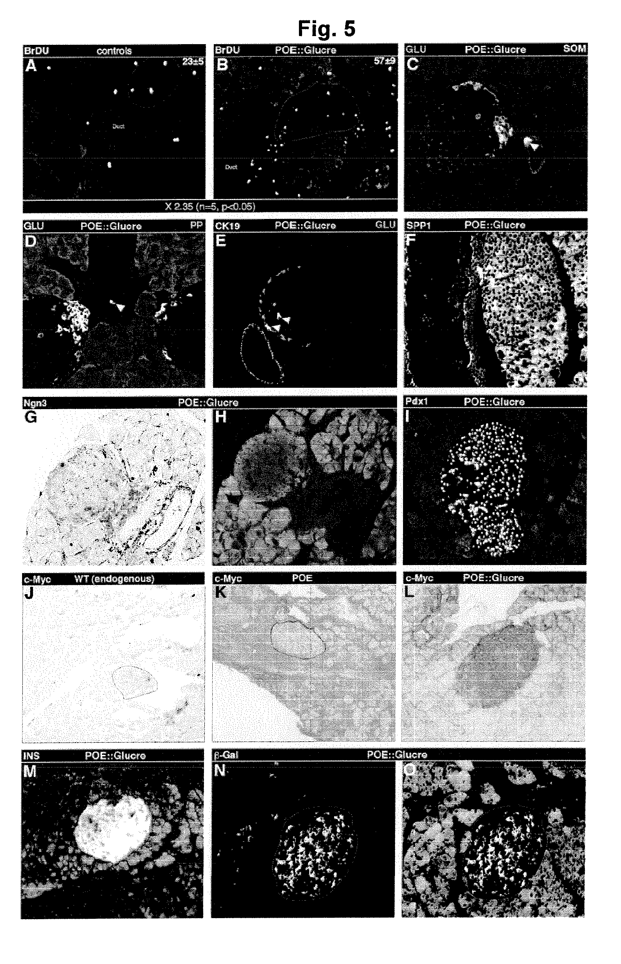

### Figure 5. Progenitor cells may be induced and converted into glucagon and subsequently into insulin-expressing cells.

Next to a pulse of BrdU at three weeks of age and examination a week later, a quantitative analysis established a 2.35 increase in the number of proliferating cells in POE::Glucre pancreas compared to POE controls (A-B). Importantly, most BrdU-labeled cells are not detected within the islets, but rather near or within the duct epithelium (B). It is worth noticing that this location corresponds to the cluster of glucagon-producing cells consistently observed in this genotype. Within the duct epithelium, scattered endocrine cells could be detected, some co-expressing the glucagon and sometimes somatostatin hormones (arrowheads in C-D). Along the same line, islet cells positive for the duct marker genes *CK19* and *SPP1* are observed adjacent to the duct epithelium (arrowheads in E-F). Notably, the duct lining also appears to contain numerous cells positive for the proendocrine marker gene *Ngn3* (G-H), but negative for *Pdx1* (I). (J-L) Infection of controls (K) and double-transgenic (L-O) pancreata using a construct containing a *CMV* promoter driving the constitutive expression of a c-Myc tag (the latter being not expressed in wild-type tissues - J). Due to the method used, two weeks post-infection, most exocrine cells are labeled by the virus, whereas only very few islet cells are (islet underlined in K). Importantly, a majority of double-transgenic endocrine cells appear marked by the virus, suggesting their ductal or acinar origin (L). These cells express the insulin hormone (M) and are β*-galactosidase*-positive (N-O), indicating that they once expressed the glucagon hormone.

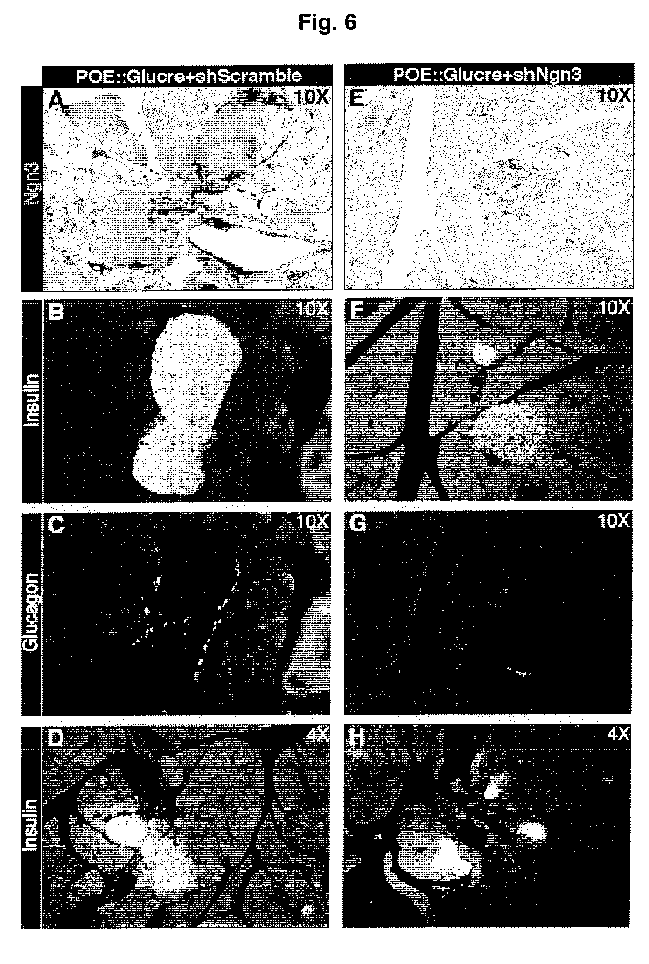

### Figure 6. Knockdown of Ngn3 prevents the Pax4-mediated β-cell hyperplasia.

(A-H) Infection of POE::Glucre animals using lentiviruses producing either an shRNA targeting *Ngn3* transcripts (E-H - Xu et al., 2008) or producing a scrambled shRNA (based on the former; A-D). Two weeks post-infection, *Ngn3* knockdown pancreata (at the indicated magnifications) display an efficient 61% diminution in Ngn3 production (Xu et al., 2008, A, E and Table 6), but also a 69% decrease in insulin- (B, F, D, H) or glucagon- (C, G) expressing cell numbers (Table 6) compared to scramble-infected counterparts.

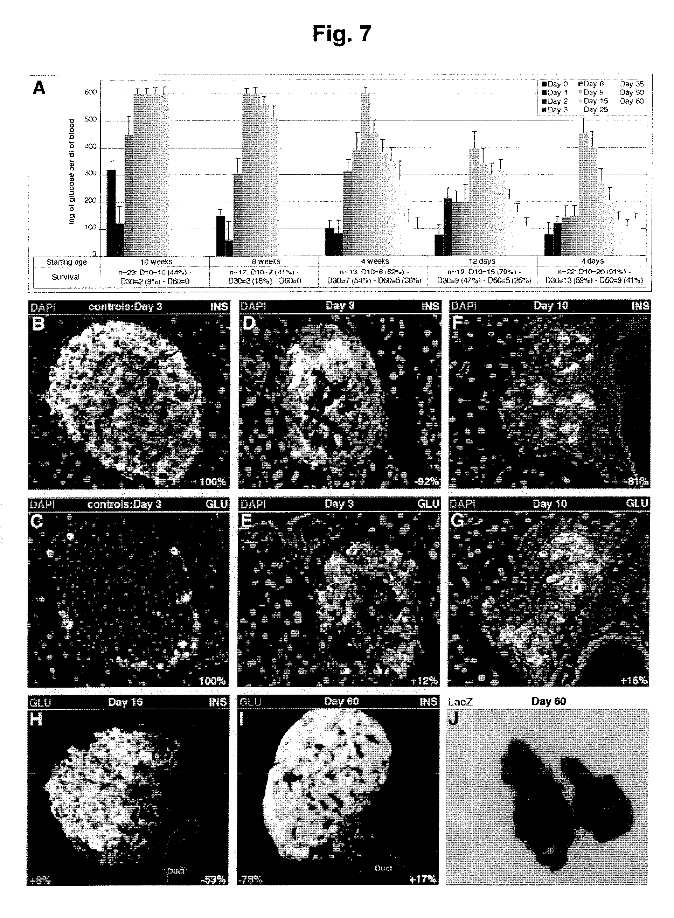

### Figure 7. Pax4 ectopic expression promotes the reconstitution of the insulin-expressing cell mass upon β-cell depletion.

(A) Following streptozotocin injection at the indicated age (starting age), the glycemia and survival of the treated animals was followed for two months. Note that animals older than four weeks of age become diabetic and die as a consequence of the obliteration of β-cells, the same being true for all controls (data not shown). Importantly, in younger animals, a steady recovery leading to normoglycemia is observed next to a peak in glucose levels (all values are expressed as means ± standard error of the mean). (B-J) Islet cell contents in 4-week-old POE::Glucre streptozotocin- (D-J) or sham- (B-C) treated animals were quantified (see at the bottom of the concerned pictures) 3 days (B-E), 10 days (F-G), 16 days (H) and 60 days (I-J) days post-injection. Three days post-injection, the β-cell mass present in controls (B-C) is almost entirely lost in streptozotocin-treated mice (D-E), the only insulin labeling being observed in areas devoid of nuclei, suggesting a detection of hormone released from killed β-cells. A 15% increase in glucagon-producing cells is highlighted 10 days post-injection compared to controls, most of these cells neighboring duct structures (G). Importantly, few insulin-labeled cells are detected (F). This trend is ascertained at day 16 (H) with a major increase in the insulin-expressing cell number. A co-detection with the glucagon hormone does not indicate any co-expression. Finally, at day 60, the β-cell mass appears statistically normal as compared to control animals (I compared to B-C), most of the cells present in the islet expressing the β-galactosidase enzyme marking glucagon-producing cells (J).

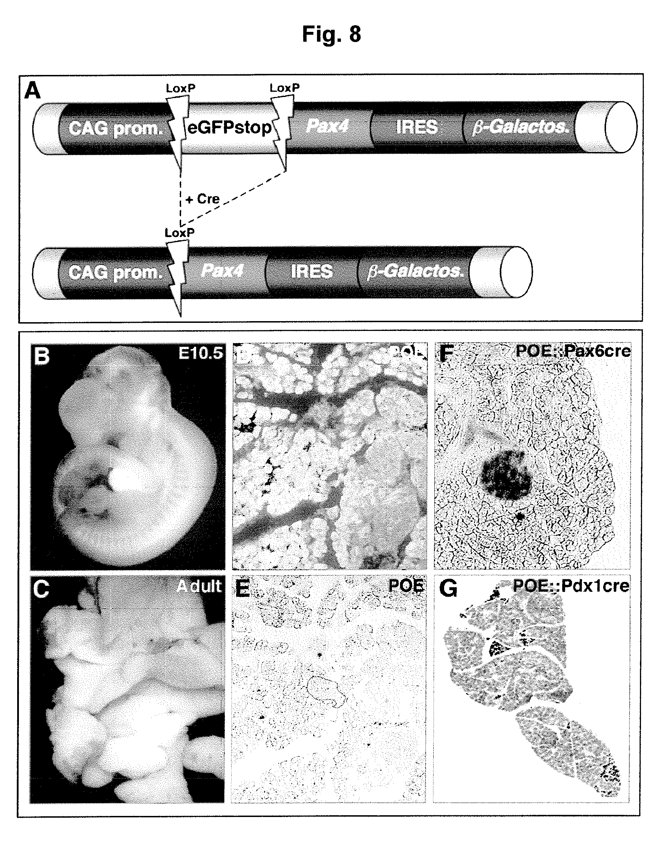

### Figure 8. Generation of animals able to conditionally and ectopically express the Pax4 gene.

(A) Schematics depicting the targeting vector prior to (top) and following (bottom) homologous recombination of the two LoxP sites induced by the phage P1 cre recombinase. β-Galactos., β-galactosidase. (B-C) Visual examination under fluorescent light of an E10.5 embryo and adult pancreas, both representative of Cre-negative animals. (D-G) POE exocrine and endocrine (note the islet outlined in D-E) cells do not express β-galactosidase as demonstrated through LacZ staining (whereas GFP staining is present in a majority of pancreatic cells; D-E). Importantly, β-galactosidase activity is found restricted to the islet of Langerhans in age-matched POE::Pax6cre pancreata (F), whereas almost all pancreatic cells are β-galactosidase-positive in POE::Pdxlcre pancreata (G).

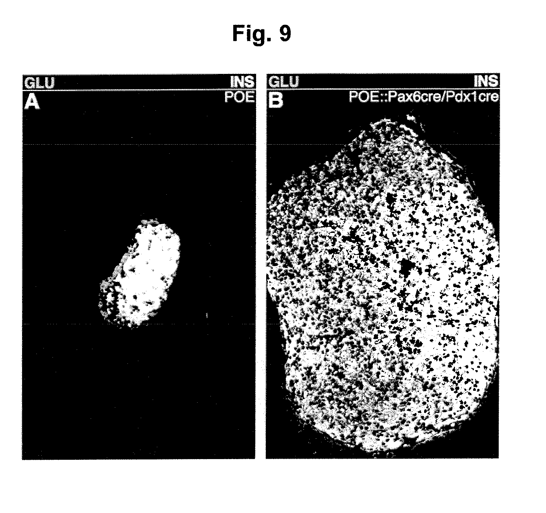

### Figure 9. The forced expression of Pax4 in Pax6 or Pdx1 expression domain results in islet overgrowth and increased β-cell mass.

Representative islets of POE (A and Figure 1E) and POE::Pax6cre/Pdxlcre (B and Figure 1G) pancreata stained with the indicated antibodies and observed using the same magnification. Note the dramatic islet size augmentation in POE::Pax6cre/Pdxlcre pancreata as a result of a drastic increase in β-cell number (B). A decrease in glucagon-expressing numbers is also highlighted in pancreata of animals ectopically expressing *Pax4* (B).

### Figure 10. The forced expression of Pax4 in Pax6 or Pdx1 expression domain results in islet overgrowth and increased β-cell mass (low magnification).

Representative sections of POE (A) and POE::Pax6cre/Pdxlcre (B) pancreata stained with anti-insulin antibodies and observed using the same magnification. Note the remarkable islet size augmentation in POE::Pax6cre/Pdxlcre pancreata (B) as a result of a drastic increase in β-cell numbers.

### Figure 11. Characterization of a newly developed anti-Pax4 antibody.

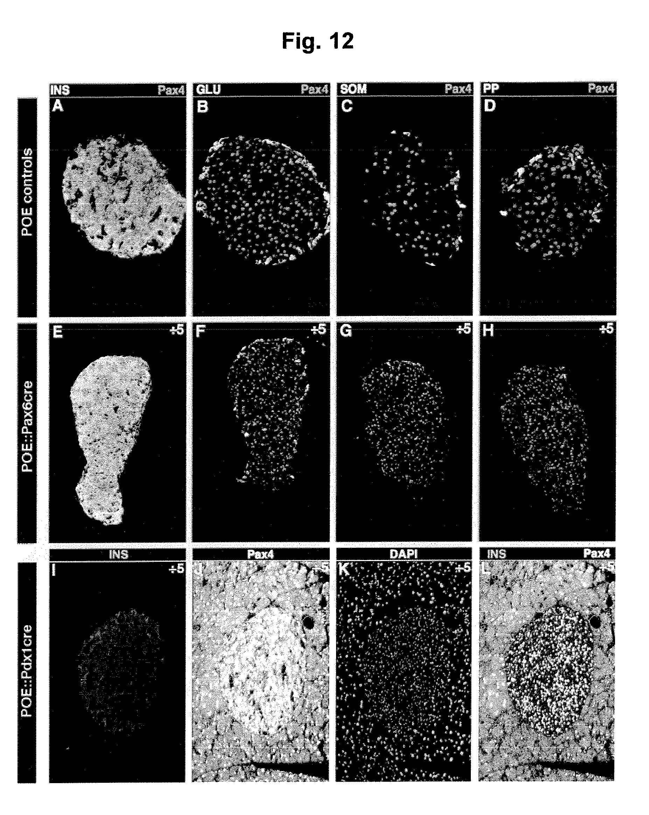

Using immunohistochemistry, an anti-Pax4 antibody (Wang et al, 2008) raised in rabbit was tested in wild-type (A-B) and Pax4-depleted (C-D) adult islets. In the wild-type situation, Pax4 is clearly detected in most β-cells (A), but not in α-cells (B), nor in δ- or PP-cells (see Figure 12). In *Pax4* mutant pancreas, characterized by a loss of β- and δ-cells at the benefit of glucagon-expressing cells, Pax4 detection is expectedly negative (C-D). The same observation was also made in *Ngn3* mutants lacking endocrine cells (data not shown). Each picture is representative of at least 3 independent animals.

### Figure 12. POE, POE::Pdxlcre and POE::Pax6cre insulin-producing cells express Pax4.

An anti-Pax4 antibody (Wang et al., 2008) was used to study *Pax4* expression in adult pancreata together with the indicated endocrine hormones. Pax4 is clearly detected in most insulin-expressing cells both in POE (A-D) and POE::Pax6cre islets (E-H), whereas α-, δ-and PP-cells are found devoid of Pax4 protein (B-D, F-H, respectively). Interestingly, the vast majority of POE::Pdxlcre pancreatic cells are found positive for Pax4 (I-L). For the purpose of clarity, the magnification of double-transgenic islets is five times reduced compared to controls. Each picture is representative of at least 4 independent animals.

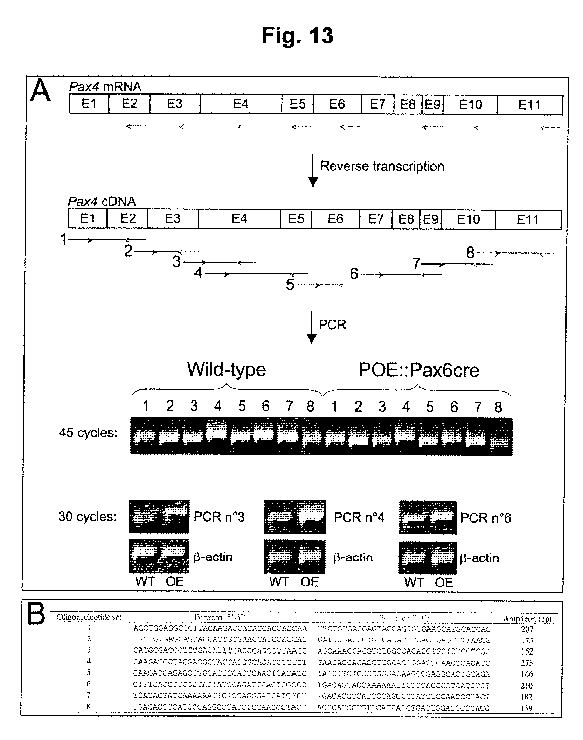

### Figure 13. Assessment of Pax4 transcripts in adult wild-type and POE::Pax6cre pancreata.

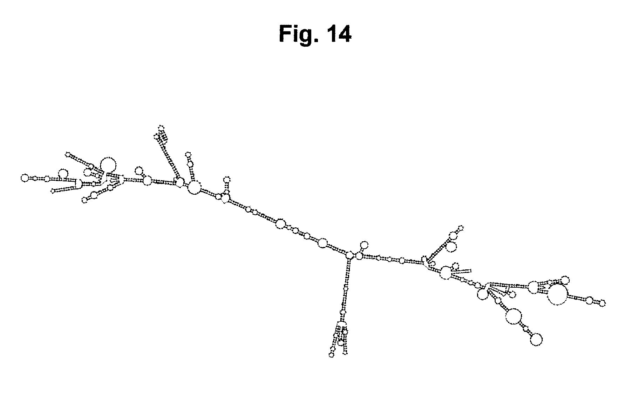

Due to conflicting reports concerning the expression of *Pax4* in adult β-cells, we attempted to amplify *Pax4* mRNA using classical RT-PCR. Such an approach was unsuccessful, most likely due to the presence of numerous secondary structures within the *Pax4* mRNA (Figure 14) and/or its weak expression. However, using a mixture of eight antisense oligonucleotides (depicted as red arrows in A) designed to cover the whole *Pax4* mRNA for the reverse transcription step (instead of classical oligo-dT oligonucleotides), subsequent PCR reactions were successful (a β-action control set being included). The location of the oligonucleotides used for these is indicated as arrows . To test whether *Pax4* transcripts were present in wild-type and POE::Pax6cre pancreata, we first used 45 cycles of elongation for the eight different PCR reactions. In both instances, similar results were obtained with a clear detection of PCR products. Semi-quantitative PCR reactions were next carried out using 30 cycles of elongation. Representative pictures are presented and demonstrate a mild expression of *Pax4* in wild-type pancreata that is strongly increased in double-transgenic animals. A list of the different oligonucleotides used is provided in B. WT, wild-type; OE, POE::Pax6cre.

### Figure 14. Pax4 mRNA predicted secondary structure.

The putative presence of secondary structures within the *Pax4* mRNA was revealed using the RNAfold program (http://mobyle.pasteur.fr/cgi-bin/MobylePortal/portal.py?form=rnafold). The plot of the predicted secondary structures calculated based on minimum free energy demonstrates that *Pax4* mRNA is exceptionally structured through matching of complementary nucleotides. This may be the cause of the difficulties encountered with classical RT-PCR, but also explains the lack of working antisense probe for *in situ* hybridization purposes. Note that this plot allows online close-up viewing.

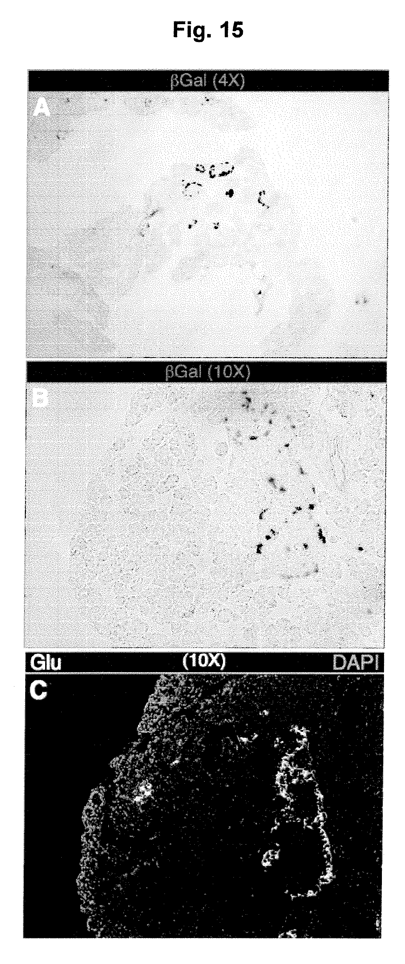

### Figure 15. Cre activity faithfully recapitulates glucagon expression in Glucre mice.

Assessment of Cre recombinase activity in glucagon promoter-Cre (Glucre - Herrera, 2000) mice crossed with Rosa26 promoter-LoxP-STOP-LoxP-β-Galactosidase reporter animals (Soriano, 1999). Cre activity was visualized using LacZ staining (A-B) at the indicated magnifications and compared to glucagon expression assayed using immunohistochemistry on a consecutive section (C). A similar expression pattern was observed for glucagon and β-galactosidase thereby confirming previously published results (Herrera, 2000). Each picture is representative of at least 3 independent animals.

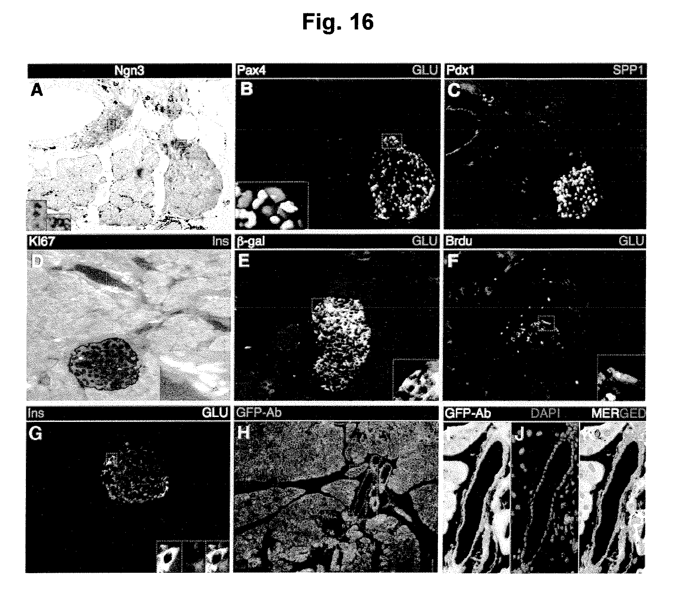

### Figure 16. Analysis of 6-week old POE::Glucre pancreata in relation to different marker genes and proliferation labels.

Serial sections of POE::Glucre animals treated with BrdU and examined a week later (F) or not were stained using the indicated antibody combinations. While Ngn3-expressing cells were found mostly located adjacent to the islet (A), Pax4- (B), Pdx1- (C) and β-galactosidase-(E) producing cells were detected exclusively within the islet of Langerhans. Only few glucagon⁺/Pax4⁺ (inlet in B) or glucagon⁺/β-galactosidase⁺ cells (inlet in E), and even fewer glucagon⁺/insulin⁺ (inlet in G), were observed in POE::Glucre pancreata, indicating an extremely rapid transition between glucagon⁺ and insulin⁺ phenotypes. Importantly, most proliferating cells were detected within the duct epithelium (D, F) or the islet domain adjacent to ducts (F). The duct epithelium and lining were found uniformly positive for GFP (H-K), suggesting that *Pax4* ectopic expression mostly occurs within the islet (B, E).

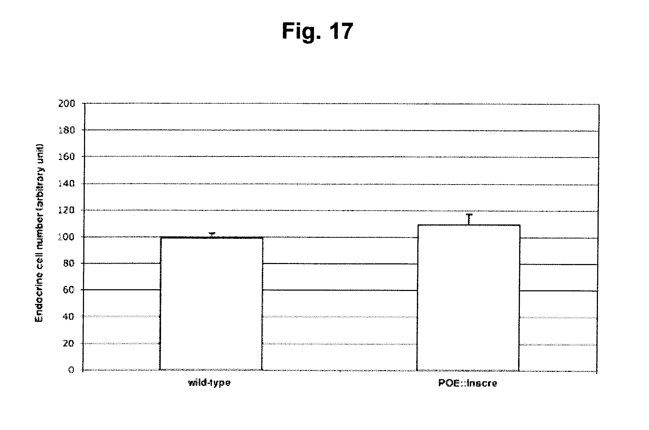

### Figure 17. An increase in Pax4 dosage does not induce β-cell proliferation.

Endocrine cell counts from adult wild-type and POE::Inscre animals were assessed after immunostaining against all four hormones. No statistically significant difference could be found between both genotypes (n=7, all values are expressed as means ± standard error of the mean).

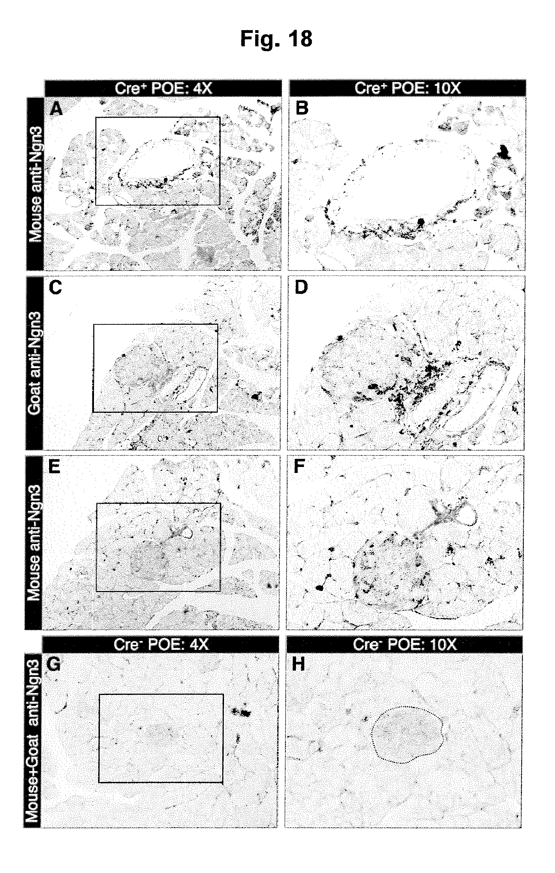

### Figure 18. Assessment of Ngn3 expression using two different anti-Ngn3 antibodies.

*Ngn3* expression was determined using a mouse anti-Ngn3 antibody (Zahn et al., 2004; A-B, E-H) and a goat anti-Ngn3 antibody (provided by M. Sander's laboratory (Seymour et al., 2007; C-D, G-H). Staining of POE (Cre⁻, G-H), POE::Pdxlcre/POE::Pax6cre/POE::Glucre (Cre⁺, A-F) pancreata suggest *Ngn3* expression in the pancreatic duct lining from animals ectopically expressing *Pax4*.

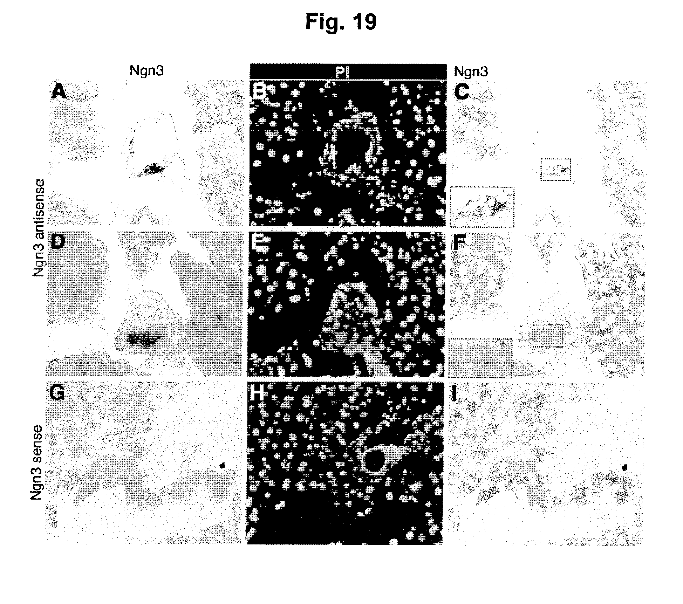

### Figure 19. Detection of Ngn3 expression in POE::Glucre pancreata using in situ hybridization.

*Ngn3* expression was assayed using two antisense probes (constructed by G. Gradwohl and P. Ravassard). (A-F) Albeit extremely difficult on adult tissues, this approach allowed us to detect few labeled cells in ductal structures in A and D (serial 18µm sections). Photographs of the same sections counterstained with propidium iodide are provided in B and E, and merged pictures in C and F, respectively. Note the expression of *Ngn3* in the cytoplasm of ductal cells (inlets in C, F). Bear in mind that due to thickness of the section (18µm) and curved nature of the duct tubes, the lumen of the latter is not always distinguishable (D-F). (G-I) Using a sense probe, we did not detect any labeled cells.

### Figure 20. Characterization of Ngn3 expression in POE::Glucre pancreata using a lentiviral approach.

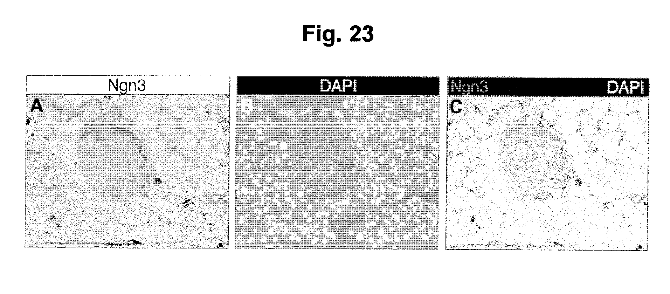

Lentiviruses encompassing a 5566-bp long fragment of the mouse *Ngn3* promoter (orthologous region of the human *Ngn3* promoter characterized in Lee et al., 2001) driving the expression of a DsRED2::c-Myc cassette (see supplementary experimental procedures) were used to infect the pancreata of POE::Glucre (C-N) or POE (A-B) animals as previously described (Xu et al., 2008). Due to the technical limitations previously discussed, the transgene was detected using an anti-c-Myc antibody. Control pancreata were found negative for c-Myc detection (A-B), whereas variable results were obtained within the same POE::Glucre pancreas, ranging from ducts negative for c-Myc (C-D), to ducts displaying different numbers of c-Myc-labeled cells (E-N), such discrepancies most likely originating from different levels of infection. Note that the specificity of the *Ngn3* promoter employed was also tested (Figure 23).

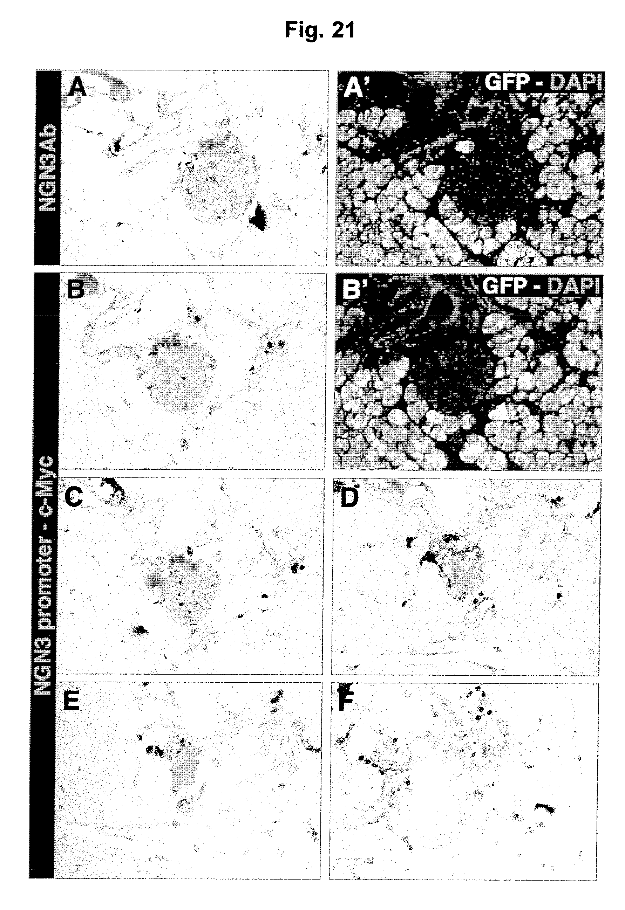

### Figure 21. Characterization of Ngn3 expression in POE::Glucre pancreata using a lentiviral approach (serial sections).

The duct displayed in Figure 20M-N was assayed on consecutive sections for c-Myc (B-F) and Ngn3 (A) expression. C-Myc-marked cells were found mostly located within the ductal lining adjacent to the islets (B-F), a locus were *Ngn3*-producing cells where detected (A). A' and B' correspond to A and B, respectively, visualized for residual GFP expression and DAPI.

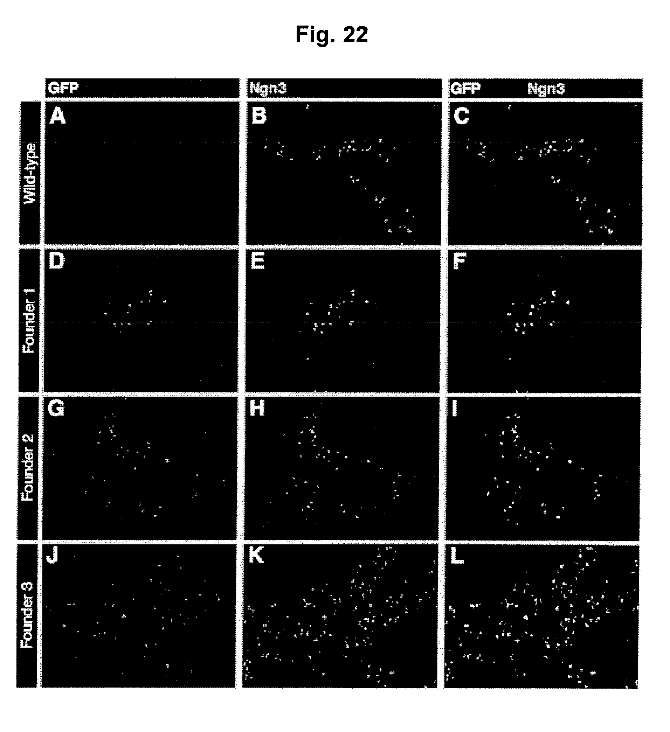

### Figure 22. Assessment of the specificity of the 5.6 kb fragment of the mouse Ngn3 promoter.

(A-L) To determine whether the 5566-bp long fragment of the mouse *Ngn3* promoter was sufficient to recapitulate *Ngn3* expression in the pancreas, a lentiviral vector expressing the *eGFP* reporter gene under the control of this promoter element was injected in the perivitelline space of mouse fertilized eggs. The resulting eggs were reimplanted in pseudo pregnant females and the derived embryos isolated 14.5 days later. Eleven transgenic founder embryos were obtained and co-stained to detect both *Ngn3* and *eGFP* expression. Briefly, using TSA amplification, 12 µm cryosections were stained with the anti-Ngn3 mouse monoclonal antibody (red, B-C, E-F, H-I, K-L) from and according to Zahn et al. (2004). Concomitantly, eGFP expression was revealed with a rabbit polyclonal anti-GFP antibody (A, C, D, F, G, I, J and L) as described by Castaing et al. (2005). (A-C) Pancreata of wild-type embryos derived from re-implanted injected eggs, are expectedly eGFP-negative (A), whereas scattered Ngn3labelled positive cells are visualized (B-C). (D-L) The analysis of representative sections of 3 pancreata of *eGFP*-expressing founders (6 out of 11) indicates that the vast majority of *Ngn3*expressing cells are eGFP positive thereby demonstrating the specificity of the 5566-bp long mouse Ngn3 promoter fragment (F, I and L).

### Figure 23. Reactivation of Ngn3 expression in glucagon-receptor-deficient pancreata.

Representative islet of adult *glucagon-receptor*-deficient animals assayed for Ngn3 (A) and counterstained with DAPI (B). A merged picture is displayed in C. Note the detection of few Ngn3-labelled cells in this genotype, whereas control age-matched counterparts are negative for Ngn3 (Figure 18G-H). A quantitative analysis of this data is presented in Table 4.

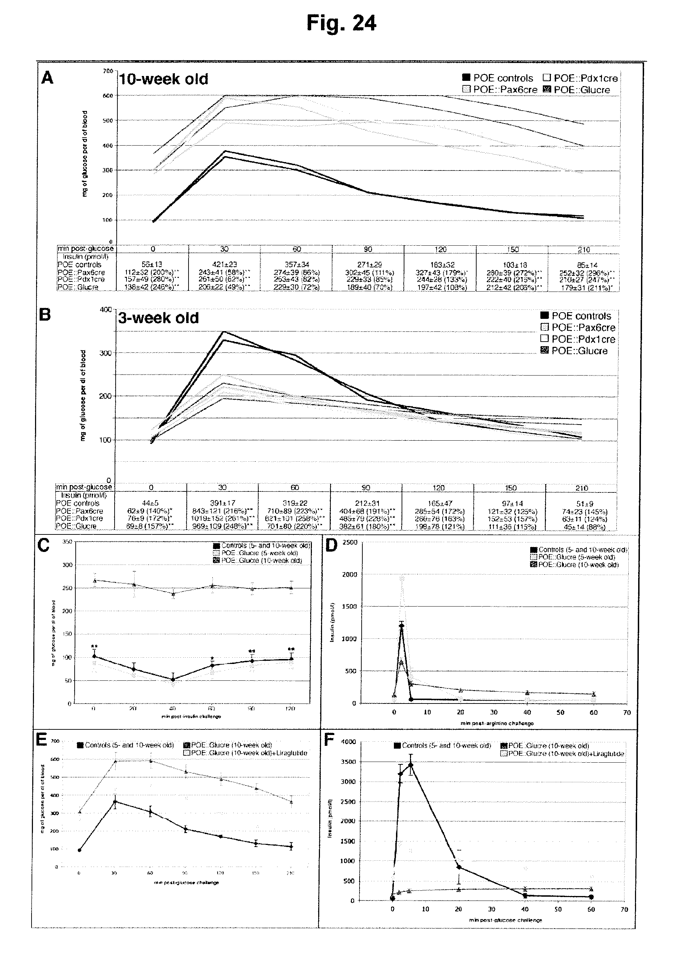

### Figure 24. In vivo assessment of β-cell function in 10-week old Pax4ectopically expressing pancreata.

(A) Through a combination of glucose challenge and circulating insulin measurements, an impairment in β-cell function is outlined in 10-week old POE::Glucre/POE::Pdxlcre/POE::Pax6cre animals: these exhibit a worsened and extended response time to glucose injection that may be explained by a decreased insulin secretion as compared to POE control counterparts. Note that 600mg of glucose per dl of blood correspond to the maximal value detectable by our glucose monitoring system. (B) For the purpose of comparison, a copy of the graph displayed in Figure 2U is included. Aiming to study the islets of double transgenic animals, these were isolated. However, due to the experimental conditions used, we noticed a disaggregating of the islets (Table 7) and therefore turned to an *in vivo* approach: (C) To determine the overall responsiveness to insulin, an insulin challenge was performed in animals of the indicated genotypes and ages. Importantly, we did not observe any significant modification of the glycemia in older POE::Glucre animals as compared to their younger counterparts or controls animals, suggesting a late-onset insulin insensitivity (n=3, **P<0.01, *P<0.05 using the lowest glycemia value within the same genotype (40 min post-challenge) as reference). (D-F) Aiming to also assay β-cell function, we treated POE::Glucre animals of the indicated genotypes and ages with two insulin secretagogues, arginine and liraglutide (a long-acting GLP-1 analog - Novo Nordisk - Denmark). It is interesting to notice that both were able to elicit an insulin secretion from hyperplastic 10-week old POE::Glucre β-cells, albeit lower than seen in younger animals (D, F). As important was the observation that liraglutide also promoted an improved glucose clearance upon glucose challenge (E), indicating that despite their altered sensitivity to glucose, older β-cells retain the ability to secrete insulin (n=3, **P<0.01, *P<0.05 using the 10-week old POE::Glucre genotype as reference).

### EXAMPLE

### INTRODUCTION

The endocrine pancreas is organized in islets of Langerhans comprising five original cell subtypes, α-, β-, δ-, ε-, and PP-cells, secreting glucagon, insulin, somatostatin, ghrelin, and pancreatic polypeptide (PP), respectively (Collombat et al., 2006). The identification and characterization of the genetic determinants underlying endocrine pancreas morphogenesis and regeneration may potentially aid designing cell replacement therapies to treat type 1 and 2 diabetes. In this context, a number of studies have demonstrated that, during development, the cooperation of several transcription factors successively specifies progenitor cells towards the pancreatic-, endocrine- and ultimately islet-cell fates. Hence, Pdx1 is required for pancreatic epithelium determination (Ahlgren et al., 1996; Ahlgren et al., 1998; Grapin-Botton et al., 2001; Jonsson et al., 1994; Offield et al., 1996) and subsequently Neurogenin3 (Ngn3) for endocrine lineage specification (Gradwohl et al., 2000; Gu et al., 2002; Jensen et al., 2000; Johansson et al., 2007). Next to Ngn3 induction, a complex network of transcription factors progressively and differentially promotes the particular endocrine fates, including Arx and Pax4 (Collombat et al., 2003; Sosa-Pineda et al., 1997). In mice lacking *Arx,* the β- and δ-cell fates were found favored at the expense of α-cell genesis, while the total endocrine cell content remained normal (Collombat et al., 2003). Conversely, in the absence of *Pax4,* the opposite phenotype was observed (Sosa-Pineda et al., 1997), indicating an inhibitory, cross-regulatory circuit between Arx and Pax4 (Collombat et al., 2005). Additional findings supported these conclusions and suggested that, firstly, Arx and Pax4 instruct endocrine precursor cells towards either an α-cell or a β-/δ-cell fate, respectively. Next, through the analysis of double-mutant mice, a secondary function of Pax4 in specifying the β-cell lineage in β-/δ-precursor-cells was uncovered (Collombat et al., 2005). Recent evidence have demonstrated that the forced expression of *Arx* in early pancreatic cells drives endocrine progenitors towards either an α- or, surprisingly, a PP-cell fate (Collombat et al., 2007). It was therefore concluded that *Arx* is not only necessary, but also sufficient to instruct the α- and PP-cell lineages.
Of particular interest was the finding that the forced expression of *Arx* triggered into adult β-cells induced their conversion into cells exhibiting α- or PP-cell phenotypes (Collombat et al., 2007). This discovery was of fundamental importance in the context of β-cell-based therapy and implied that the opposite conversion might be achieved, that is, to generate β-cells from other endocrine cells. To test this hypothesis, we generated mice conditionally and ectopically expressing the *Pax4* gene. Our data indicates that the ectopic expression of *Pax4* in early pancreatic cells, but also in α-cells, induces their respecification towards a β-cell fate or identity. As a consequence of the ensuing glucagon deficiency, an ongoing neogenesis of α-cells occurs. However, such α-cells are continuously converted into β-cells upon *Pax4* ectopic expression, resulting in the development of oversized islets of Langerhans. Importantly, a prominent expression of the proendocrine transcription factor Ngn3 in the pancreas of such animals is highlighted. Our results are consistent with the recently reported notion of facultative adult stem cells that reactivate *Ngn3* expression in injured pancreas (Xu et al., 2008). Finally, following streptozotocin-induced depletion of β-cells in young mice ectopically expressing *Pax4,* an α-cell-mediated regeneration of the β-cell mass, a progressive normalization of the glycemia and an extended lifespan are observed.

### RESULTS

### Pax4 ectopic expression in the developing pancreas promotes the genesis of oversized islets mainly composed of β-cells

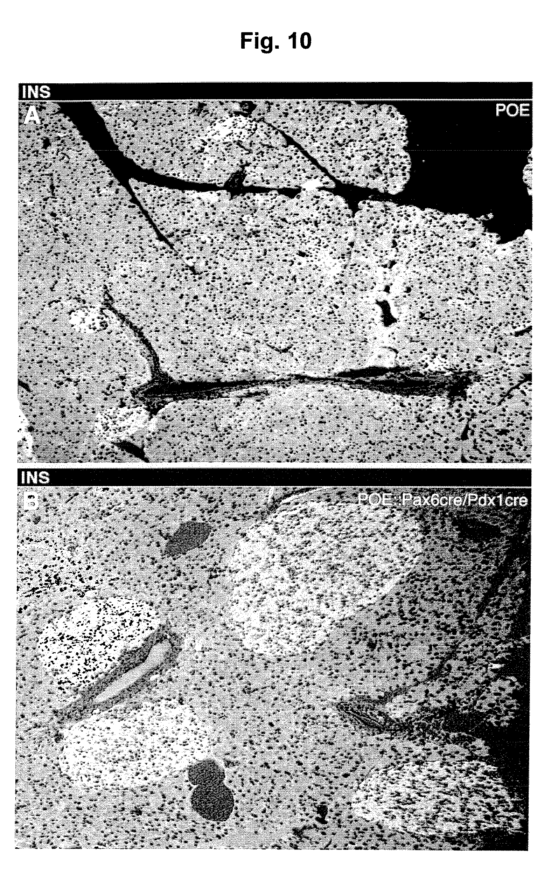

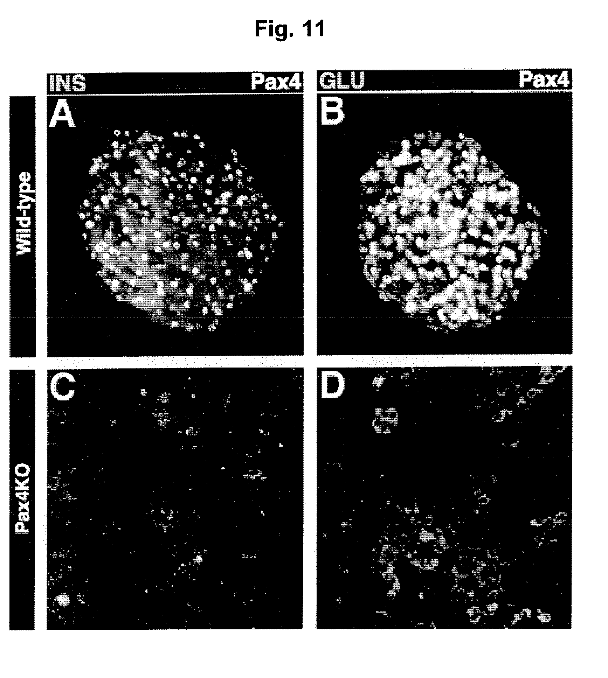

Taking advantage of the Cre-LoxP system, we generated transgenic mice able to conditionally express the *Pax4* gene (POE). Briefly, the construct used included the cytomegalovirus enhancer/human β-actin (CAG) promoter driving the expression of the *eGFP* gene followed by translation stop codons in all three frames and flanked by LoxP sites (Figure 8A). A *Pax4* cDNA-IRES-β-galactosidase sequence was subsequently cloned downstream of *eGFP.* Using pronuclear injection, five independent transgenic mouse lines were derived. The resulting mice constitutively expressed *eGFP,* but not β galactosidase, at all ages examined (Figure 8B- E).
POE animals were bred with Pdx1 cre or Pax6cre animals to target *Pax4* expression to the pancreatic epithelium or the endocrine tissue, respectively (Ashery-Padan et al., 2004; Gu et al., 2002). Double-transgenic POE::Pdxlcre and POE::Pax6cre mice were born and developed normally. However, animals of both genotypes eventually died at the age of 3 to 12 weeks, a majority after 9 weeks (Figure 1A and data not shown). To assess whether the ectopic expression of *Pax4* altered glucose homeostasis, blood sugar levels were measured. One day *postpartum,* a decrease in glycemia was evidenced in both POE::Pdxlcre and POE::Pax6cre genotypes (Figure 1A). Notably, at six weeks of age, blood glucose levels were found normal in these same animals compared to controls, whereas shortly before death (SBD), a dramatic hyperglycemia was uncovered. This indicates that POE::Pdxlcre and POE::Pax6cre mice initially present a hypoglycemic condition that progressively evolves towards a hyperglycemic one.
To assess the consequences of the forced expression of *Pax4* in *Pdx1* and *Pax6* expression domains, immunohistochemical analyses of double-transgenic pancreata were performed. As similar results were obtained in POE::Pax6cre and POE::Pdxlcre mice, pictures representative of both genotypes are displayed for 6-week-old animals (Figure 1C, F, I, L, O) and SBD (Figure 1D, G, J, M, P). We did not observe any change in endocrine cell composition in POE animals as compared to their wild-type counterparts (Figure 1B, E, H, K, N and data not shown). The analysis of both POE::Pax6cre and POE::Pdxlcre pancreata showed a dramatic increase in islet size (note the 2 and 8-fold scale reduction in magnification in Figure 1C, F, I, L, O and D, G, J, M, P, respectively - Figure 9-10) containing mainly insulin-expressing cells (Figure 1F-G, O-P, Figure 9-10) and only few δ- and Arx-labeled α- and PP-cells (Figure 1F-G, I-J, L-M, O-P). Notably, the remaining α-, δ- and PP- cells were consistently located at one pole of the islet of Langerhans (Figure 1F-G, I-J, L-M, O-P), a subset of these co-expressing the glucagon and somatostatin or PP hormones (arrows in Figure 11, L, respectively). A quantification of these alterations (Figure 1Q - Table 1) ascertained (1) an age-dependent increase in islet size and β-cell mass, concurrently with a reduction in non-β-cell numbers (relative as well as absolute numbers), and (2) an abnormal location of the few remaining α-, δ- and PP-cells at one pole of the islet.
*Pax4* expression was assayed with a newly-developed antibody that detected Pax4 in wild-type embryonic pancreata (Wang et al., 2008) and specifically in adult β-cells (Figure 11A-B and 12A-D), as previously reported (Theis et al., 2004). Neither in *Pax4*-depleted, nor in *Ngn3*-deficient islets, did we find any expression of Pax4 (Figure 114C-D and data not shown). Importantly, in POE::Pax6cre mice, most insulin-producing cells were found positive for Pax4 (Figure 12E-H), whereas the vast majority of pancreatic cells were labeled in POE::Pdxlcre animals (Figure 125I-L). These results were confirmed by RT-PCR analysis (Figure 136) and assessment of β-galactosidase activity (Figure 8F-G).
Further examination of POE::Pax6cre and POE::Pdxlcre mice during the development of the endocrine pancreas indicated that α-, δ, and PP-cell lineages were disfavored to the benefit of an insulin-producing cell fate (Figure 2A-H). Most insulin-labeled cells present in double-transgenic mice correctly expressed the β-cell specific markers Nkx6.1, Pdx1, Glut2 and HB9 (Figure 2K-P and data not shown). Accordingly, they lacked Arx (Figure 2I-J) and Brn4 (Figure 2Q-R), labeling α-/PP- and α-cells, respectively, whereas Pax6 and Isl1 were detected in all islet cell types, as expected (Figure 2S-T and data not shown). To further assess the function of these insulin-producing cells postnatally, glucose tolerance tests associated with systemic insulin measurements were performed. Consistent with the increased β-cell mass we previously noted, three-week-old POE::Pdxlcre and POE::Pax6cre mice displayed an improved glucose clearance associated with a more than two-fold increase in circulating insulin levels (Figure 2U). Altogether, our findings suggest that the ectopic expression of the *Pax4* gene drives endocrine precursor cells almost exclusively towards an insulin-expressing cell fate, and that these cells exhibit a functional β-cell phenotype.

### Conversion from an α- to a β-cell phenotype

As Pax4 specifically favors the β-cell fate and identity throughout the morphogenesis of the endocrine tissue, we wondered whether this might also apply to mature endocrine cells. Therefore, animals conditionally expressing *Pax4* in glucagon-producing cells (Herrera, 2000 - Figure 15) were generated (POE::Glucre) and endocrine cell numbers were monitored. As early as one week *postpartum,* a 50% enlargement in islet size was evidenced, these containing increased numbers of insulin⁺/Pax4⁺ cells compared to controls (Figure 3A). Notably, the content of glucagon-producing cells was found reduced by 77% and most of the remaining glucagon⁺ cells were located at one pole of the islet (Figure 3A-C). As expected, δ- and PP-cell numbers were unchanged (Figure 3B-C). It is worth noticing that the expression of *Pax4* in glucagon-producing cells was accompanied by the production of the β-galactosidase protein (Figure 81A) allowing lineage-tracing experiments. One week following birth, this otherwise glucagon-cell-specific labeling was detected in the majority of insulin-producing cells (Figure 3D-F) expressing a typical β-cell complement of transcription factors (Table 2). Very few cells were found positive for both insulin and glucagon (Figure 169G). Besides, we observed an age-dependent increase in islet size and in the number of insulin-/β-galactosidase-producing cells, the latter exhibiting most β-cell features (Table 2-3, Figure 3G-I, 2U). This suggests that, upon *Pax4* ectopic expression, adult glucagon-expressing cells are continuously converted into cells exhibiting a β-cell phenotype.

### Pax4 ectopic expression in β-cells does not induce their proliferation

To further characterize the origin of the supernumerary β-cells, we assayed proliferating cells combining bromodeoxyuridine (BrdU) incorporation and Ki67 staining. Following a BrdU pulse in 5-week old POE::Glucre mice, pancreata were quantitatively analyzed a week later: a significant 2.35-fold increase in the number of BrdU-labeled cells was evidenced as compared to controls (Figure 5A-B, Table 4). Interestingly, most BrdU⁺ (Figure 5B-16F), but also Ki67⁺ (Figure 16D) cells were located outside the islets, within or adjacent to the neighboring duct epithelium.
The cell-autonomous effect of an augmentation of the Pax4 dose in β-cells was analyzed by crossing POE with Inscre mice expressing the cre recombinase under the control of the insulin promoter (Herrera, 2000). The quantification of the endocrine cell numbers in 3-week old POE::Inscre pancreata did not reveal any increase in β-cell numbers, nor in islet size (Figure 17 and data not shown), despite the use of the POE mouse line inducing the highest *Pax4* expression (Table 5, line 5). Thus, although Pax4 may promote a modest proliferation of β-cells in rat islet cultures (Brun et al., 2004), alternative mechanisms clearly act to boost the robust expansion of the β-cell mass consistently observed in POE::Pdx1cre, POE::Pax6cre and POE::Glucre mice (from hereon termed "double-transgenic animals"). One issue of concern to our approach was related to the overexpression of *Pax4.* However, through the use of different POE transgenic founder mice, we demonstrated that the ectopic expression of *Pax4* at a dose lower than found in normal β-cells, is sufficient to induce a loss of the α-cell phenotype to the benefit of β-cell features (Table 5).

### Rescuing glucagon deficiency prevents the generation of oversized islets

As lineage-tracing experiments demonstrated that glucagon-expressing cells were converted into cells displaying a β-cell phenotype, a depletion of α-cells was expected. However, we consistently detected clusters of glucagon⁺ cells in our double-transgenic mice (Figures 1-3), suggesting that α-cell neogenesis occurred. We therefore hypothesized that the latter might be driven by the hypoglucagonemia resulting from the α- to β-cell conversion. To test this theory, we injected 3-week-old POE::Pdx1Cre, POE::Glucre, POE::Pax6cre, and control animals twice daily with glucagon during three weeks. A significant decrease in islet size was observed in glucagon-treated animals as compared to controls (Figure 4). This was mainly attributed to a reduction in the β-cell mass (Figure 4E, G compared to C, and K compared to I, Table 3-4). Importantly, the number of α-cells was also found significantly diminished (Figure 4F, H, L and Table 3-4). These results thus indicate that decreased glucagon levels are responsible for the continuous replenishment of α-cells that acquire a β-cell phenotype in double transgenic animals.

### Requirement of Ngn3 re-expression for α-cell neogenesis and ensuing acquisition of a β-cell phenotype

The location of endocrine non-β-cells at one pole of the islet of Langerhans of double-transgenic animals was remarkable. This observation was confirmed through z-stack analysis of POE::Pdx1Cre, POE::Glucre and POE::Pax6cre whole islets stained with anti-glucagon antibodies (data not shown). Most importantly, these glucagon-labeled cell clusters were consistently found adjacent to the ductal lining (data not shown). The very detection of such glucagon⁺ cells was intriguing as their conversion into β-cells was expected upon *Pax4* expression. A likely explanation may be the continuous generation of glucagon-labeled cells, their detection indicating a transitional state prior to their conversion into insulin-expressing cells. This hypothesis was supported by the active cell proliferation observed near and within the duct epithelium (Figure 5B, 16D, F), close to the pole of glucagon⁺ cells. In addition, scattered glucagon⁺ cells were found within the lining of the duct (arrowheads in Figure 5C-D), whereas the duct markers cytokeratin-19- (CK19) and SPP1 were detected not only within the duct epithelium, but also in few adjacent islet cells (arrowheads in Figure 5E-F). The existence of facultative progenitor cells in adult mice that, under specific injury conditions, reactivate *Ngn3* expression and develop into all four endocrine cell types has been recently reported (Xu et al., 2008). Interestingly, in pancreata ectopically expressing *Pax4,* we also noticed a reactivation of the islet cell progenitor marker Ngn3 (Figure 5G-H, 16A, 18-19, Table 3-4), while *Pax4* expression was never detected in duct cells (Figure 16B). However, due to the difficulties classically encountered to detect Ngn3 in adult tissues, the definitive location and counts of Ngn3-producing cells will await the generation of better antibodies/*in situ* probes. We therefore asked whether *Ngn3* reactivation was involved in the generation of supernumerary insulin⁺ cells by tracing exocrine- and Ngn3-producing cells, or inhibiting *Ngn3* expression in the pancreas of double transgenic mice. Because the expression of Cre, GFP and β-galactosidase in our double-transgenic animals precluded the use of duct-Cre, Ngn3-Cre, Ngn3-GFP or Ngn3-LacZ mouse lines for tracing purposes, a lentiviral approach was preferred. Specifically, recombinant lentiviruses constitutively expressing a c-Myc-tagged DsRED2 reporter were injected into the pancreatic duct of POE::Glucre mice, as previously described (Xu et al., 2008). Due to the high intensity of the GFP signal present in some animals, native DsRED2 fluorescence could not be unambiguously ascertained. We therefore employed immunohistochemistry to detect the exogenous c-Myc epitope-tag fused to DsRED2 (the endogenous c-Myc being unrecognized - Figure 5J). Two weeks post-infection, Myc-tagged cells were evidenced in the vast majority of duct and acinar cells of control animals, whereas less than 5% of endocrine cells were labeled, as reported previously (Xu et al., 2008 - Figure 5K). In the pancreas of double-transgenic mice, however, 71% of endocrine cells were positive for the Myc tag (Figure 5L), most of them expressing insulin (Figure 5M) and β-Galactosidase (Figure 5N-O), the latter labeling cells that previously expressed *glucagon*. This result suggests that exocrine cells were converted into endocrine cells in the double transgenic mice. Double transgenic mice were also infected with recombinant lentiviruses expressing either the c-Myc-tagged reporter under the control of the *Ngn3* promoter or a *Ngn3*-specific short hairpin (sh) interfering RNA controlled by the *CMV* promoter, as in Xu et al. (2008). Transduction with the first construct both confirmed the re-expression of *Ngn3* and the specificity of *Ngn3* promoter employed (Figure 20-22). Interestingly, *Ngn3* knockdown caused a dramatic reduction in the number of Ngn3⁺ (Figure 6A, E), insulin⁺ (Figure 6B, F, D, H) and glucagon⁺ (Figure 6C,G) cells, as well as in the level of transcripts encoding *Ngn3, insulin* and *glucagon* (Table 6).
To test whether glucagon signaling deficiency may also induce the reactivation of *Ngn3,* in addition to POE::Glucre animals, glucagon receptor-deficient mice (characterized by an α-cell hyperplasia - Gelling et al., 2003) were analyzed. Combining Real-time RT-PCR and counts of immunostained sections (Table 3-4, Figure 23), an increase in *Ngn3,* but also in the β-cell regeneration-associated *Reg3b* (or *INGAP -* Rosenberg, 1998) gene expression was observed in both genotypes. Interestingly, *Ngn3* and *Reg3b* transcripts as well as Ngn3⁺ cell numbers appeared dramatically reduced in POE::Glucre animals supplemented with glucagon for three weeks (Table 3-4). Thus, our data supports the notion that the glucagon deficiency mediated by the ectopic expression of *Pax4* results in *Ngn3* expression and in the successive conversion of progenitor cells into glucagon- and thereafter insulin-producing cells.

### Pax4 ectopic expression rescues streptozotocin-induced diabetes.

Thus far, our data suggested that the forced expression of *Pax4* in either *Pax6, Pdx1* or *glucagon* expression domains ultimately led to the development of oversized islets of Langerhans mostly composed of seemingly differentiated and functional β-cells. We reasoned that such cells might be capable of replacing lost β-cells in diabetic mice. Hence, POE::Glucre mice of different ages were injected with a single dose of the β-cell toxin streptozotocin (STZ), and both blood glucose levels as well as viability were monitored for 8 weeks (Figure 7A). Shortly after STZ injection, we often observed a decrease in glycemia, most likely caused by the discharge of insulin from killed β-cells (Figure 7A). A higher mortality rate was found among animals older than 4 weeks, as compared to younger mice. It should be noticed that these animals were already hyperglycemic and weakened prior to STZ injection due to a combined insulin insensitivity and β-cell failure to optimally respond to a glucose challenge (Figure 24). In contrast, in 4-week-old and younger STZ-treated animals, we noted a normalization in blood glucose levels following a peak in glycemia and survival rates reaching 41 % two months post-injection (Figure 7A), while all controls died (data not shown). Immunohistochemical analyses demonstrated that following the rapid obliteration of the β-cell mass (Figure 7B-E), a massive neogenesis of glucagon-expressing cells occurred, again near the duct lining (Figure 7F-I). Ten days post-injection, only few insulin-producing cells were detected (Figure 7F-G). However, a steady increase in glucagon- and insulin-expressing cell numbers was observed in the following days (Figure 7H). Approximately two months post-injection, POE::Glucre animals exhibited almost normally-sized islets of Langerhans and normoglycemia (Figure 7I). Interestingly, at that age, the β-galactosidase lineage tracer was found uniformly distributed in islet cells (Figure 7J), demonstrating that the insulin-producing cells in these animals derived from cells that previously expressed *glucagon.* These insulin⁺ cells displayed a β-cell phenotype and expressed a β-cell-specific complement of transcription factors (data not show). Altogether, our analyses provide direct evidence that, in STZ-treated mice, the ectopic expression of *Pax4* in α-cells continuously converts them into β-cells and counters diabetes in animals younger than four weeks of age.

### DISCUSSION

In this study, we report that the forced expression of the *Pax4* gene in the mouse endocrine pancreas results in oversized islets composed mainly of cells displaying a β-cell phenotype. Our findings are consistent with the induction of progenitor cells that adopt an α-cell identity as a consequence of decreased glucagon levels, and subsequently acquire β-cell features upon *Pax4* ectopic expression, *Ngn3* reactivation being instrumental in this processes. The resulting β-cells are functional at least at an early age, and can repopulate the islets of diabetic mice to normalize blood sugar levels.

### Pax4 promotes the β-cell fate specification during embryogenesis and induces an α-cell-mediated β-cell neogenesis postpartum

The forced expression of *Pax4,* either in Pdx1⁺ pancreatic progenitor cells (and ultimately in all pancreatic cells) or in Pax6⁺ endocrine precursor cells (and ultimately in all islet cells) results in the nearly exclusive specification of cells exhibiting a β-cell identity at the expense of the α-, δ- and PP-cell lineages. At birth, these animals display normal pancreas morphology, unaffected exocrine tissue and well-sized islets of Langerhans, indicating that the main alterations observed are solely related to the allocation of the different endocrine cell lineages during embryonic development. This also suggests that Pax4 does not alter the pancreatic exocrine differentiation program, but rather acts on the specification of endocrine progenitor cells by promoting the acquisition of the β-cell fate. Interestingly, following birth, an age-dependent increase in islet size is evidenced in pancreata ectopically expressing *Pax4* that was attributed to the continuous generation of cells displaying a β-cell phenotype. In an effort to ascertain the origin of such cells, lineage-tracing experiments were performed using POE::Glucre mice. Our initial goal was to trigger the ectopic expression of *Pax4* in adult glucagon-producing cells, but an inducible glucagon-cre line is hitherto unavailable. Hence, the classical glucagon-cre mouse line (Herrera, 2000) was used to induce *Pax4* expression in glucagon-producing cells. It is important to notice that during embryogenesis, the *glucagon* gene is initially expressed in early endocrine cells often co-expressing additional hormones, including insulin. However, by irreversibly tagging the progeny of cells using the Cre/LoxP system, Herrera (2000) demonstrated that mature glucagon- and insulin-producing cells do not derive from cells that previously expressed insulin or glucagon, respectively. Interestingly, lineage-tracing experiments performed in POE::Glucre mice revealed that the vast majority of newly formed β-galactosidase⁺/insulin⁺ cells originate from cells that previously expressed *glucagon* (note that endogenous β-galactosidase⁻/insulin⁺ cells remain detectable). Based on these results, but also on the age-dependent increase of β-galactosidase⁺ β-cell numbers and the concomitant decrease in α-cell contents, the dramatic expansion of the β-cell mass throughout the postnatal lifespan of double transgenic mice was attributed to a continuous neo-formation of β-cells through α-cell redifferentiation rather than to the slow self-renewal capacity of β-cells (Dor et al., 2004). Although our findings are in agreement with a putative neogenesis/conversion of somatostatin- or PP-positive cells into β-cells, testing this hypothesis will have to await the generation of somatostatin-cre and PP-cre mice to allow lineage-tracing experiments.

### Ngn3 is required for the continuous neogenesis of α-cells ultimately acquiring a β-cell phenotype upon Pax4 ectopic expression.

We demonstrate that glucagon supplementation reduces the β-cell hyperplasia in double-transgenic mice, most likely by compensating the deficiency in circulating glucagon resulting from the loss of α-cells through Pax4-induced redifferentiation. Interestingly, compromised glucagon signaling has previously been associated with α-cell neogenesis: both *Glucagon receptor (Gcgr)-* and *prohormone convertase 2 (Pcsk2)-*deficient animals display oversized islets, mainly composed of glucagon-producing cells (Furuta et al., 1997; Gelling et al., 2003). Similar to the present report, exogenous glucagon treatment significantly reduced the endocrine cell hyperplasia in *Pcsk2*-deficient animals (Blume et al., 1995; Petersson and Hellman, 1963; Webb et al., 2002). *Pcsk2* mutants were also found to contain glucagon⁺ cells near the duct epithelium and their contribution to the neogenesis of the supernumerary glucagon⁺ cells was suggested. Accordingly, our findings indicate that, upon *Pax4* ectopic expression, a physiologically significant glucagon deficiency activates a continuous compensatory response resulting in α-cell neogenesis, as seen in *Gcgr* and *Pcsk2* mutant mice. However, these cells are subsequently converted into β-cells upon *Pax4* ectopic expression.
In animals ectopically expressing *Pax4,* but also in *Gcgr* mutants, a reactivation of *Ngn3,* a gene normally exclusively expressed during embryonic development of the pancreas and required for the endocrine differentiation program, was observed. However, due to the difficulties encountered to detect Ngn3 transcripts or protein, the determination of the origin of such Ngn3⁺ cell will require more elaborated lineage-tracing experiments and/or more specific antibodies/*in situ* probes. Interestingly, lentivirus-mediated cell tracing and knockdown experiments showed that *Ngn3* re-expression is in fact crucial for the α-cell-mediated β-cell neogenesis. As important was a recent report demonstrating that, upon pancreatic duct ligation, facultative adult stem cells are activated along the lining of duct epithelium (Dor and Melton, 2008; Xu et al., 2008). It was also established that these cells reactivate *Ngn3,* differentiate into endocrine cells and contribute to the formation of oversized islets. In mice ectopically expressing *Pax4,* our results suggest that duct-lining cells may represent the source of the neogenerated glucagon-expressing cells. Although we cannot exclude the contribution of acinar cells to this process, additional evidence favor a duct-to-islet cell conversion mechanism: (1) the continuous detection of neo-generated glucagon⁺ islet cells adjacent to duct structures, (2) the active proliferation within the duct epithelium and near the islet pole where non-β endocrine cells accumulate, (3) the presence of glucagon⁺ endocrine cells in the ductal lining, and (4) the detection of cells expressing duct markers within the islet. Our findings are supported by recent reports demonstrating the plasticity of pancreatic cells. For instance, D. Melton and coworkers proved that acinar cells could be reprogrammed into β-cells upon the ectopic expression of selected genes *in vivo* (Zhou et al., 2008), whereas Inada *et al.* (2008) provided evidence that duct cells may give rise to endocrine and acinar cells in the adult pancreas. In agreement with these, our analysis underlines that hypoglucagonemia activates compensatory mechanisms provoking the conversion of progenitor cells into α-cells that subsequently acquire a β-cell phenotype upon *Pax4* ectopic expression, these Ngn3-dependent processes ultimately leading to the generation of oversized islets of Langerhans.

### Pax4 ectopic expression rescues from streptozotocin-induced diabetes.

We reasoned that the continuous β-cell neogenesis observed in double-transgenic animals might be able to rescue experimentally-induced diabetes. Therefore, the function of the newly formed β-cells was assessed in diabetic mice with more than 95% β-cell loss following STZ treatment. No insulin supplement was used to counter the sudden β-cell loss, as the consequences of exogenous insulin treatment on islet cells formation are hitherto unclear. Accordingly, a high lethality was expected, especially in older hyperglycemic and weakened mice. However, while all control and double transgenic mice older than 10 weeks died, 41% of the younger animals survived during two months post STZ injection. Closer examination revealed a progressive reconstitution of the islets and normalization of the glycemia and indicated that the new insulin⁺ cells behaved as true β-cells. Also under these conditions, clusters of glucagon⁺ cells were detected at one pole of the islets, adjacent to duct structures, such cells subsequently adopting a β-cell phenotype. Furthermore, the endocrine tissue in animals that died during the course of these experiments showed significant β-cell neogenesis, but, most likely, not sufficient to allow survival.
The observation that only double transgenic animals younger than four weeks of age could survive STZ treatment was intriguing. Interestingly, this age corresponds to the period when such animals, initially hypoglycemic, progressively develop a hyperglycemic condition. While the low blood glucose levels observed after birth can be easily explained by the β-cell hyperplasia, the development of a diabetic condition was unexpected: the steady increase in insulin-producing cell content and the reduced number of glucagon-expressing cells are in contrast with the observed high glucose levels. In the light of the data reported in this study, it appears that the newly-formed insulin-expressing cells are functional for at least four weeks following birth and respond normally to a glucose challenge. During this time, they express all the β-cell markers we tested and are negative for α-, δ- and PP-cell marker genes. However, glycemia, glucose tolerance, insulin secretion and insulin sensitivity deteriorate in older animals despite a normal expression of a typical β-cell complement of transcription factors, suggesting that older β-cells fail to trigger an optimal response upon glucose challenge. Interestingly, these retain some capacity to secrete insulin when challenged with arginine or a long-acting GLP-1 analog. The reasons for the establishment of such a condition are unclear, but may result from progressive (1) β-cell exhaustion, (2) β-cell alterations, (3) desensitiza-tion of the insulin receptor/pathway as a consequence of increased insulin levels, and/or (4) unknown effects induced by the decreased glucagon/somatostatin/PP hormone contents. Defining the mechanisms involved would require further work in which the impact of β-cell hyperplasia, but also of the decrease in α-, δ- and PP-cells contents, could be modulated and analyzed independently. It is interesting to notice that younger double-transgenics subjected to streptozotocin treatment display an extended lifespan in comparison to untreated counterparts (data not shown). This indicates that the development of the diabetic condition in these mice is not age-related, but may rather depend on the islet hyperplasia state. Clearly, using different activation times of exposure to Pax4 would allow us to determine its long-term effect on islet function. Together, our results suggest that the sole ectopic expression of *Pax4* in glucagon⁺ cells can, in younger animals, reverse the consequences of streptozotocin-mediated diabetes through the induction of α-cell neogenesis and their ensuing conversion into functional β-cells. Based on these findings, we suggest that drug-mediated modulation of Pax4 and/or its targets may open new avenues for treatment of diabetes and, in addition, may contribute to strategies aiming to differentiate β-cells from stem, progenitor or other cell types.

### EXPERIMENTAL PROCEDURES

### Mouse Manipulations

The strategy used to generate the POE mouse line is depicted is Figure 8. These mice were crossed with Pdx1-, Pax6-, Glucagon and Insulin-cre lines (Ashery-Padan et al., 2004; Gu et al., 2002; Herrera, 2000) and genotyped using a combination of fluorescence microscopy for GFP examination and genotyping PCR for *cre* and β*-galactosidase* genes.
To assess the effects of glucagon on islet size, mice were injected intraperitoneally twice daily (every 12h) with 5µg of glucagon and sacrificed after 3 weeks of treatment. For STZmediated diabetes induction, a freshly prepared 50mg/ml solution in 0.1mol/l sodium citrate, pH 4.5 was injected intraperitoneally (200mg/kg). Lentivirus production and injection were performed as described previously (Xu et al., 2008). Lastly, BrdU was injected intraperitoneally (200µl of 100µg/ml) and was detected by immunohistochemistry (Invitrogen).

### Immunohistochemistry

Tissues were fixed in 4% PFA overnight at 4°C, embedded in paraffin and 8-µm sections applied to slides. These sections were assayed as described previously (Collombat et al. 2003). In order to perform co-immunofluorescence, the GFP signal was bleached when necessary, as described (Collombat et al., 2007). The primary antibodies used were the following: mouse monoclonal anti-insulin, anti-glucagon (1/1000 - Sigma), anti-Ngn3 (1/2000- 1F25A1B3, BCBC Antibody Core), anti-c-Myc (1/100 - Abcam); guinea pig anti-insulin, anti-glucagon (1/1000 - Sigma); rabbit anti-somatostatin (1/600 - Dako), anti-PP (1/200 - Dako), anti-Nkx6.1 (1/3000), anti-Nkx2.2 (1/1000 - kindly provided by T. Jessell), anti-Pax6 (1/500 - Chemicon), anti-Pax4 (1/500), and anti-Arx (1/1000); chicken anti-β-galactosidase (1/500 - Biozol); goat anti-Ngn3 (1/1000 - kindly provided by M. Sander). The secondary antibodies (1/1000 - Molecular Probes) used were: 594-alexa anti-mouse; 488-alexa anti-mouse; 594-alexa anti-rabbit; 488-alexa anti-rabbit; 594-alexa anti-guinea pig; 488-alexa anti-guinea pig. Pictures were processed using confocal microscopy. For quantification purpose, stained cells were counted manually on every tenth section and the count reported to the pancreatic area estimated *in silico.*

### Glucose Challenge and Circulating Glucose or Insulin Level Measurements

For glucose challenge tests, 6 mice per genotype were fasted for 24h and injected intraperitoneally with glucose (2g/kg) and blood glucose levels measured 0, 30, 60, 90, 120, 150, and 210min afterwards. At each time point, one animal per genotype was sacrificed immediately after glycemia assessment for serum insulin level determination using RIA (Linco). Glucose levels (mg/dl) were determined with the One Touch Glucose monitoring kit (Johnson & Johnson).

### Data Analysis

All values are depicted as mean ± standard error of the mean from at least three independent experiments and considered significant if p < 0.05. All data were statistically analyzed by multivariate comparison (two-way ANOVA) with Bonferroni correction or one-way ANOVA with Newman-Keuls correction.

### RNA Analysis

RNA isolation (RNAeasy, Qiagen) and cDNA synthesis (Supercript choice system, Invitrogen) were performed according to the manufacturer's instructions. cDNA synthesis for analyses aiming to detect *Pax4* transcripts was performed using a mixture of eight *Pax4*-derived (and β-actin-derived) antisense oligonucleotides instead of the provided oligo-dT primers (Figure 13B).
Quantitative RT-PCR were carried out using the QuantiTect SYBR Green RT-PCR Kit (Qiagen) and validated primers (Qiagen) according to the manufacturer's instructions. The PCR reactions and detection were performed on a Mastercycler® ep realplex cycler using GAPDH and HPRT1 as internal controls for normalization purposes.

### Lentiviral vector construction to specifically target Ngn3-expressing cells

A 5566-bp long fragment of the *Ngn3* promoter spanning from position -5286 to +280 relative to transcription start site was amplified by PCR from a genomic lambda clone containing the mouse *Ngn3* locus using the high fidelity Phusion DNA polymerase (FINNZYMES) with the following primers: pNgn3 Fw =
5' CACCAAGCTTTGTGTGGAAGGAAATGTC and pNgn3 Rv =
CGCGCGCCCCTCATCCACCCTTTG. The resulting PCR product was cloned into the pENTR/D-Topo plasmid (Invitrogen) and the insert sequenced to rule out PCR-mediated mutations. The resulting plasmid was used to perform LR Clonase II (Invitrogen) *in vitro* recombination in order to insert the *Ngn3* promoter fragment upstream of either DSRed2::c-Myc or eGFP reporters in the pTrip deltaU3 lentiviral backbones. The lentiviral vector stocks were produced as described before (Castaing 2005).

### Production of transgenic mice using lentiviral-mediated gene transfer

The lentiviral vector expressing eGFP under the control of the 5566-bp long fragment of the Ngn3 promoter was injected into the perivitelline space of mouse fertilized eggs as described previously (Lois et al. 2002). The injected eggs were next re-implanted into pseudo pregnant females and the resulting embryos isolated 14.5 days afterwards. They were genotyped with eGFP specific primers
(Fw = 5' GACGTAAACGGCCACAAGTTC 3' and
Rev = 5' GTCGCCCTCGAACTTCACCTC 3') and fixed in 4% PFA overnight, cryoprotected in 15% sucrose PBS and frozen. Cryo-sections were stained for eGFP (Castaing et al., 2005) and Ngn3 (Zahn et al., 2004), and counterstained with DAPI.

### In situ hybridization

For RNA *in situ* hybridization, pancreata were collected, fixed, incubated overnight in 30% sucrose, embedded in cryomatrix, and 18-µm sections were applied to Probe-on Plus slides (Fisher Scientific). Defrosted sections were hybridized overnight with DIG-labeled probes in a medium containing 50% formamide, 10% dextran sulphate, 1 mg/mL yeast tRNA, 0.02% BSA, 0.02% Ficoll, and 0.02% PVP. Tissues were washed successively in 50% formamide, 1X SSC, 0.1% Tween-20 at 70°C for 70 min, and MABT at pH 7.5 (100mM maleic acid; 150mM NaCl; 0.1% Tween-20) at room temperature for 1 h and blocked in PBS containing 20% inactivated fetal calf serum for 90 min. Anti- DIGantibody (1:2500) was applied overnight in the same solution at room temperature. Tissues were washed thoroughly in MABT for 2 h, rinsed with NTMT (100 mM NaCl; 100 mM Tris-HCl at pH 9.5; 50 mM MgC12; 1% Tween-20) for 1 h, stained in a solution containing 350 µg/mL NBT, 175 µg/mL BCIP, rinsed in PBS, and fixed in 4% paraformaldehyde.

### Insulin, arginine and liraglutide tolerance tests

Animals of the indicated genotypes were injected subcutaneously with Actrapid insulin (0.75 mU/g - Novo Nordisk - Denmark), intravenously with arginine (5mg - Sigma) or subcutaneously with liraglutide (200 micrograms per kg - Novo Nordisk - Denmark). Following insulin challenge, blood glucose levels were measured as described in the main manuscript. Next to arginine challenge, at each time point, one animal per genotype was sacrificed immediately for serum insulin level determination using RIA (Linco). Lastly, in the case of liraglutide, the latter was injected 30 min prior to glucose challenge. At each time point, three animals per genotype were sacrificed immediately for serum glucose and insulin level determination.

### TABLES

Quantification of the endocrine contents in POE::Pdxlcre and POE::Pax6cre pancreata (average cell counts per square centimetre of pancreas)

**Table 1. Quantification of the endocrine cell content alterations in the pancreata of POE::Pdxlcre and POE::Pax6cre animals.**

| | | POE/ WT | POE::Pdx1cre | POE::Pax6cre |
|---|---|---|---|---|
| | Insulin | 127±9 | 165±19 (+29%)** | 171±12 (+35%)** |
| | | | | |
| | Glucagon | 43±8 | 9±1 (-79%)** | 6±1 (-86%)** |
| P24h | | | | |
| | Somatostatin | 25±6 | 11±2 (-56%)** | 10±2 (-60%)** |
| | | | | |
| | PP | 8±2 | 2±0 (-75%)* | 6±1 (U) |
| | Insulin | 278±33 | 1245±101 (+350%)*** | 1022±99 (+267)*** |
| | | | | |
| | Glucagon | 93±23 | 27±9 (-70%)** | 22±10 (-76%)** |
| 6 | | | | |
| weeks | | | | |
| | Somatostatin | 62±12 | 31±13 (-50%)** | 36±14 (-42%)** |
| | | | | |
| | PP | 9±2 | 5±3 (U) | 3±1 (-66%)* |
| | Insulin | 299±56 | 1702±169 (+470%)*** | 1799±125 (+501%)*** |
| | | | | |
| Short | Glucagon | 88±22 | 14±6 (-84%)** | 9+7(-90%)** |
| before | | | | |
| death | Somatostatin | 49±8 | 12±5 (-76%)** | 14±8 (-72%)** |
| | | | | |
| | PP | 18±7 | 3±2 (-83%)* | 2±1 (-89%)* |

A quantitative endocrine marker analysis was performed on pancreata of the indicated genotypes and ages. Using immunohistochemical detection, hormone-expressing cells were counted on every tenth section of pancreas and reported to the pancreatic area estimated *in silico.* The values presented correspond to the averages of hormone-producing cells per square centimeter (also reported in Figure 1Q). Note the age-dependent increase in the β-cell mass at the expense of the other endocrine cell subtypes. (n>4, ***P<0.001, **P<0.01, * P<0.05).

| | | | | | | |
|---|---|---|---|---|---|---|
| *Pax4* ectopic expression promotes glucagon-expressing cells to acquire a β-cell phenotype | | | | | | |

| Age | E20 | | 6 weeks | | 12 weeks | |
|---|---|---|---|---|---|---|
| | *POE* | *POE: : Glucre* | *POE* | *POE: : Glucre* | *POE* | *POE: : Glucre* |
| Insulin | 133±12 | 176±9 (+24%) | 267±20 | 1633±187 (+512%) | 299±29 | 2764±356 (+824%) |
| | | | | | | |
| β-galactosidase | 1±2 | 51±13 | *0* | 1444±167 | *0* | 2612±289 |
| | | | | | | |
| Glucagon | 58±6 | 15±7 (-73%) | 83±13 | 17±11 (-79%) | 111±45 | 12±9(-89%) |
| | | | | | | |
| Somatostatin | 21±4 | 17±5 (U) | 35±8 | 31±14 (U) | 42±12 | 40±19 (U) |
| | | | | | | |
| PP | 3±1 | 2±0 (U) | 8±6 | 7±5 (U) | 10±9 | 8±7 (U) |
| | | | | | | |
| Arx | 74±10 | 18±9 (-74%) | 99±10 | 36±12 (-64%) | 114±18 | 24±15 (-79%) |
| | | | | | | |
| Nkx6.1 | 129±14 | 172±13 (+25%) | 259±21 | 1672±223 (+545%) | 258±31 | 2865±401 |
| | | | | | | |
| Pdx1 | 130±10 | 164±9 (+21%) | 253±33 | 1567±201 (+518%) | 279±32 | 2537±505 (+810%) |
| | | | | | | |
| Glut-2 | 128±8 | 177±8 (+28%) | 241±33 | 1616±157 (+570%) | 276±44 | 2969±356 (+974%) |
| | | | | | | |

**Table 2. Quantification of the endoerine cell content alterations in the pancreata of POE::Glucre animals.**

| | | | | | | |
|---|---|---|---|---|---|---|
| HB9 | 126±11 | 171±11 (+26%) | 252±28 | 1644±335 (+552%) | 319±48 | 2748±287 (+760%) |
| | | | | | | |
| Brn4 | 66±9 | 14±6 (-78%) | 78±9 | 22±14 (-72%) | 107±23 | 19±4 (-82%) |
| | | | | | | |
| Nkx2.2 | 187±18 | 192±11 (U) | 334±41 | 1439±201 (+330%) | 425±41 | 2714±188 (+538%) |
| | | | | | | |
| Isll | 214±18 | 208±19 (U) | 385±50 | 1757±176 (+356%) | 453±30 | 3152±667 (+595%) |
| | | | | | | |
| Pax6 | 216±25 | 203±31 (U) | 390±38 | 1886±331 (+383%) | 460±28 | 3146±491 (+584%) |
| | | | | | | |
| Islet area | 100% | 99.8% | 100% | +381.1% | 100% | +583.2% |
| | | | | | | |
| Glucose levels | - | - | 91mg/dl | 121mg/dl (+33%) | 101mg/dl | 351mg/dl (+248%) |
| | | | | | | |
| Life expectancy | normal | 3-12weeks | normal | 3-12weeks | normal | 3-12weeks |

A quantitative endocrine marker analysis was performed on sections of pancreata of the indicated genotypes and ages using immunohistochemical detection, hormone-expressing cells were counted on every tenth section of pancreas and reported to the pancreatic area estimated *in silico.* The values presented correspond to the averages of positive cells per square centimetre of pancreas (n>3, P<0.05, except when labeled with U, Unchanged). Note the loss of α-cells and the concomitant increase in the number of β-galactosidase⁺ insulin-producing cells and associated markers (Nkx6.1, Pdx1, Glut2, Isl1, HB9) in double transgenic animals, concurrently with an increase in the total endocrine cell number.

**Table 3. Real time RT-PCR analysis of selected transcripts in 3- and 6-week old pancreata of different genotypes.**

| | | | | |
|---|---|---|---|---|
| Real time RT-PCR analysis of selected transcripts in animals of different genotypes | | | | |

| | 3week old animals | | 6week old animals | |
|---|---|---|---|---|
| | POE::Glucre | GluR-KO | POE::Glucre | POE::Glucre +glucagon |
| Insulin | 423±24% | 37±9% | 559±82% | 285±52% |
| | | | | |
| Glucagon | 22±11% | 1350±98% | 27±15% | 18±9% |
| | | | | |
| Pax4 | 608±98% | 41±4% | 514±87% | 555±91% |
| | | | | |
| Ngn3 | 1244±89% | 623±44% | 1120±159% | 475±81% |
| | | | | |
| Reg3b | 623±22% | 1070±109% | 724±93% | 434±63% |
| | | | | |
| Arx | 27±6% | 1480±121% | 17±9% | 15±10% |
| | | | | |
| Pdx1 | 274±39% | 31±8% | 502±85% | 279±62% |
| | | | | |
| Nkx6.1 | 398±51% | 40±4% | 491±60% | 183±20% |

The expression of the indicated genes was assayed in pancreatic tissues of POE::Glucre, POE::Glucre supplemented with glucagon, and *glucagon-receptor* mutant (GluR-KO) mice using age-matched tissues of WT and POE pancreata as reference (values set at 100%). POE::Glucre, but also GluR-KO pancreata display increased contents of *Ngn3* and *Reg3b* transcripts. Additionally, an augmentation in β-cell-specific transcripts is outlined in POE::Glucre, whereas the expression of the α-cell marker genes is reduced. Importantly, concomitantly with a significant decrease in *Ngn3* and *Reg3b* transcript contents, a reduction in insulin- and glucagon-producing cell numbers (and associated marker genes) is evidenced in POE::Glucre animals supplemented with glucagon. All values are statistically significant (P<0.05, n>2) versus POE/WT.

**Table 4. Assessment of cell contents in 3- or 6-week old pancreata of different genotypes.**

| | | | | | | |
|---|---|---|---|---|---|---|
| Quantification of cell numbers expressing selected genes in animals of different genotypes (average cell counts per square centimetre of pancreas) | | | | | | |

| | | 3week old animals | | | 6week old animals | |
|---|---|---|---|---|---|---|
| | POE/WT | POE::Glucre | GluR-KO | POE/WT | POE::Glucre | POE::Glucre +glucagon |
| Insulin | 182±71 | 821±107** | 59±33* | 267±20 | 1103±128** | 394±132**^{##} |
| | | | | | | |
| Glucagon | 72±21 | 32±26 | 789±137** | 83±13 | 19±6** | 6±4**^{#} |
| | | | | | | |
| Ngn3 | - | 53±9 | 21±8 | - | 63±15 | 38±11^{#} |
| | | | | | | |
| BrdU (1 week) | - | - | - | 23±5 | 57±9** | 28±7^{#} |

The counts in cells positive for the indicated genes are reported per square centimetre of pancreatic tissues of POE::Glucre, POE::Glucre supplemented with glucagon, and glucagon-receptor mutant (GluR-KO) mice using age-matched tissues of WT/POE pancreata as reference. It is worth noticing that, in animals ectopically expressing *Pax4* in *glucagon* expression domains or in GluR-KO mice, Ngn3-producing cells reemerge (note for the latter that due to the bias introduced by the high background, this count should be considered as indicative). However, their content becomes significantly reduced in POE::Glucre mice supplemented with glucagon. In the latter case, a concomitant decrease in BrdU-labelled proliferating cell numbers is highlighted (BrdU provided at 5weeks of age, examination a week later). (n>2, **P<0.01, *P<0.05 using the POE/WT genotype as reference; ^{##}P<0.01, ^{#}P<0.05 using the POE::Glucre genotype as reference).

Quantification of transcript contents in the five different POE::Glucre mouse lines

**Table 5. The ectopic expression of Pax4 is sufficient to induce alterations in the endocrine cell content.**

| | POE- -Line 1 to 5- | POE::Glucre -Line 1- | POE::Glucre -Line 2- | POE::Glucre -Line 3- | POE::Glucre -Line 4- | POE::Glucre -Line 5- |
|---|---|---|---|---|---|---|
| Insulin | 100% | +383% | +352% | +417% | +336% | +399% |
| | | | | | | |
| Glucagon | 100% | -91% | -89% | -96% | -79% | -93% |
| | | | | | | |
| Somatostatin | 100% | U | U | U | U | U |
| | | | | | | |
| PP | 100% | U | U | U | U | U |
| | | | | | | |
| Pax4 | 100% | **+457%** | +834% | +2006% | **+339%** | +2776% |

In order to verify a putative relationship between phenotypic alterations and *Pax4* expression levels, we crossed the five different POE founder lines with Glucre animals. A similar outcome was observed in all cases, resulting in an increase in the insulin-expressing cell content at the expense of the glucagon-producing cell number. Using a combination of GFP intensity examination and real-time PCR analysis, different levels of *Pax4* expression were highlighted in these lines (see thereafter), most probably due to distinct chromosomal integration loci of the transgene. In 3-week-old wild-type mice, *Pax4* is normally produced in β-cells representing 68.21% of endocrine cells. In age-matched double-transgenics, a quantitative analysis demonstrated an expression of *Pax4* in 93.35% of islet cells. Hence, we postulated that, if *Pax4* was to be expressed at the same physiological dosage in double transgenic pancreata as in wild-type, the global *Pax4* pancreatic dosage found in controls should then be relatively increased 1.37-fold (93.35/68.21) in double-transgenic pancreata. However, one also needs to factor in the total endocrine cell number increase upon expression of *Pax4* in α-cells. A careful quantitative analysis demonstrated a 3.67 augmentation of the islet cell count between controls and double-transgenic animals at that age. Thus, the expected increase in *Pax4* dosage, assuming a similar cellular *Pax4* concentration across genotypes, should be of 5.02 (1.37X3.67). Interestingly, in two of our transgenic lines (lines 1 and 4, in bold-face and underlined), the global pancreatic increase of *Pax4* expression was found lower than this threshold with values of 4.57 and 3.39, respectively. However, even in these animals, severe alterations in islet cell content were evidenced, indicating that, not only the overexpression of *Pax4,* but also its ectopic expression in glucagon-producing cells, is able to induce a loss of the α-cell phenotype at the profit of β-cell features. All reported values are statistically significant (P<0.05, n=3) and were further confirmed by immunohistochemistry (data not shown).

**Table 6. Ngn3 knock-down induces a decrease in the insulin-expressing cell hyperplasia observed in POE::Glucre mice.**

| Assessment of the effect of *Ngn3* knock-down in POE::Glucre animals | | | | |
|---|---|---|---|---|
| | Transcript relative expression | | Absolute cell count/cm² | |
| | POE::Glucre+Scramble | POE::Glucre+shRNA | POE::Glucre+Scramble | POE::Glucre+shRNA |
| Ngn3 | 100±10% | 33±7%** | 55±12 | 21±7 (-61%) ** |
| | | | | |
| Insulin | 100±4% | 68±8%* | 1340±172 | 402±61 (-70%) ** |
| | | | | |
| Glucagon | 100±12% | 43±11%* | 23±7 | 7±5(-69%)* |
| | | | | |
| Reg3b | 100±13% | 44±16%** | - | - |

Animals of POE::Glucre genotype were infected with lentiviruses containing a shRNA either targeting Ngn3 production or a scrambled version. A clear decrease in *Ngn3* and *Reg3b* transcript contents and Ngn3⁺ cell counts was observed two weeks post-infection (note for the latter that due to the bias introduced by the high background, this count should be considered as indicative). Concurrently, insulin- or glucagon-expressing cells as well as insulin or glucagon transcript contents were found significantly reduced.

**Table 7. Perifusion analysis of isolated POE: :Glucre isolated islets.**

| | | | | |
|---|---|---|---|---|
| Assessment of isolated islet properties using perifusion in POE::Glucre animals | | | | |
| | | | | |

| | % of insulin released reported to the total insulin cell content/2h | | | |
|---|---|---|---|---|
| Glucose stimulation: | 2.5mM | 7.5mM | 20mM | 20mM+glucagon |
| 3-week old | | | | |
| | | | | |
| Control | 0.7±0.2 | 0.8±0.1 | 8.2±0.2 | 8.9±1.1 |
| | | | | |
| POE::Glucre | 0.8 | 0.8 | 9.4 | 10.6 |
| | | | | |
| 12-week old | | | | |
| | | | | |
| Control | 0.7±0.1 | 1.0±0.3 | 10.4±1.7 | 9.5±0.8 |
| | | | | |
| POE::Glucre | 0.5±0.0 | 0.7±0.3 | 6.9±0.7 | 6.6±1.2 |

Islets of 4- and 12-week-old POE::Glucre mice were isolated by handpicking, following collagenase digestion (0.3 mg/ml) of the pancreata, to study their secretory capacity in a glucose perifusion assay as described by Stangé et al. (2003). Unfortunately, we failed to detect *Pax4* ectopically expressing oversized islets, suggesting that these were overdigested, presumably because of differences in cell-cell adhesion, known to be crucial for islet structure and function, between control and double transgenic mice (Rogers GJ et al., Cell Physiol Biochem, 2007; Caton D et al, Diabetes Metab, 2002; Pipeleers, Experientia, 1984). Obviously, a decrease in digestion time/collagenase concentrations would not have permitted proper islet isolation. We anyway perifused the islets and observed no difference in islet function of younger animals for all the genotypes analyzed. However, in older counterparts, we did detect a decrease in the glucose-stimulated insulin release, albeit not significant. It should be noticed that such a lack of significance might be attributed to the loss of oversized islets induced by experimental conditions.

### REFERENCES

Ahlgren, U., Jonsson, J., and Edlund, H. (1996). The morphogenesis of the pancreatic mesenchyme is uncoupled from that of the pancreatic epithelium in IPF1/PDX1-deficient mice. Development 122, 1409-1416.
Ahlgren, U., Jonsson, J., Jonsson, L., Simu, K., and Edlund, H. (1998). beta-cell-specific inactivation of the mouse Ipf1/Pdx1 gene results in loss of the beta-cell phenotype and maturity onset diabetes. Genes Dev 12, 1763-1768.
Ashery-Padan, R., Zhou, X., Marquardt, T., Herrera, P., Toube, L., Berry, A., and Gruss, P. (2004). Conditional inactivation of Pax6 in the pancreas causes early onset of diabetes. Dev Biol 269, 479-488.
Blume, N., Skouv, J., Larsson, L.I., Holst, J.J., and Madsen, O.D. (1995). Potent inhibitory effects of transplantable rat glucagonomas and insulinomas on the respective endogenous islet cells are associated with pancreatic apoptosis. J Clin Invest 96, 2227-2235.
Breinbauer R, Manger M, Scheck M, Waldmann H. (2002) Natural product guided compound library development. Curr. Med. Chem. 9(23):2129-2145
Brun, T., Franklin, I., St-Onge, L., Biason-Lauber, A., Schoenle, E.J., Wollheim, C.B., and Gauthier, B.R. (2004). The diabetes-linked transcription factor PAX4 promotes {beta}-cell proliferation and survival in rat and human islets. J Cell Biol 167, 1123-1135.
Brutlag (1990) Comp. App. Biosci. 6:237-245
Castaing M, Guerci A, Mallet J, Czernichow P, Ravassard P, and Scharfmann (2005) Efficient restricted gene expression in beta cells by lentivirus-mediated gene transfer into pancreatic stem/progenitor cells. Diabetologia 48: 709-719.
Caton D et al, Diabetes Metab, 2002.
Collombat, P., Hecksher-Sorensen, J., Serup, P., and Mansouri, A. (2006). Specifying pancreatic endocrine cell fates. Mech Dev 123, 501-512.
Collombat, P., Hecksher-Sorensen, J., Broccoli, V., Krull, J., Ponte, I., Mundiger, T., Smith, J., Gruss, P., Serup, P., and Mansouri, A. (2005). The simultaneous loss of Arx and Pax4 genes promotes a somatostatin-producing cell fate specification at the expense of the alphaand beta-cell lineages in the mouse endocrine pancreas. Development 132, 2969-2980.
Collombat, P., Hecksher-Sorensen, J., Krull, J., Berger, J., Riedel, D., Herrera, P.L., Serup, P., and Mansouri, A. (2007). Embryonic endocrine pancreas and mature beta cells acquire alpha and PP cell phenotypes upon Arx misexpression. J Clin Invest 117, 961-970.
Collombat, P., Mansouri, A., Hecksher-Sorensen, J., Serup, P., Krull, J., Gradwohl, G., and Gruss, P. (2003). Opposing actions of Arx and Pax4 in endocrine pancreas development. Genes Dev 17, 2591-2603.
Dor, Y., Brown, J., Martinez, O.I., and Melton, D.A. (2004). Adult pancreatic beta-cells are formed by self-duplication rather than stem-cell differentiation. Nature 429, 41-46.
Dor, Y., and Melton, D.A. (2008). Facultative endocrine progenitor cells in the adult pancreas. Cell 132, 183-184.
Gene Transfer, Delivery and Expression of DNA and RNA, A Laboratory manual, Edit by T. Friedmann, J. Rossi, Cold Spring Harbor Laboratory Press, New York 2006
Furuta, M., Yano, H., Zhou, A., Rouille, Y., Holst, J.J., Carroll, R., Ravazzola, M., Orci, L., Furuta, H., and Steiner, D.F. (1997). Defective prohormone processing and altered pancreatic islet morphology in mice lacking active SPC2. Proc Natl Acad Sci U S A 94, 6646-6651.
Gelling, R.W., Du, X.Q., Dichmann, D.S., Romer, J., Huang, H., Cui, L., Obici, S., Tang, B., Holst, J.J., Fledelius, C., et al. (2003). Lower blood glucose, hyperglucagonemia, and pancreatic alpha cell hyperplasia in glucagon receptor knockout mice. Proc Natl Acad Sci U S A 100, 1438-1443.
Gradwohl, G., Dierich, A., LeMeur, M., and Guillemot, F. (2000). neurogenin3 is required for the development of the four endocrine cell lineages of the pancreas. Proc Natl Acad Sci U S A 97, 1607-1611.
Grapin-Botton, A., Majithia, A.R., and Melton, D.A. (2001). Key events of pancreas formation are triggered in gut endoderm by ectopic expression of pancreatic regulatory genes. Genes Dev 15, 444-454.
Gu, G., Dubauskaite, J., and Melton, D.A. (2002). Direct evidence for the pancreatic lineage: NGN3+ cells are islet progenitors and are distinct from duct progenitors. Development 129, 2447-2457.
Herrera, P.L. (2000). Adult insulin- and glucagon-producing cells differentiate from two independent cell lineages. Development 127, 2317-2322.
Hitt MH, Philipp Ng, and Graham FL (2006) Construction and Propagation of Human Adenovirus Vectors. Cell Biology, a laboratory handbook; third edition, Vol 1, chapter 53, pp 435-443.
Hussain, M.A., Lee, J., Miller, C.P., and Habener, J.F. (1997). POU domain transcription factor brain 4 confers pancreatic alpha-cell-specific expression of the proglucagon gene through interaction with a novel proximal promoter G1 element. Mol Cell Biol 17, 7186-7194.
Inada, A., Nienaber, C., Katsuta, H., Fujitani, Y., Levine, J., Morita, R., Sharma, A., and Bonner-Weir, S. (2008). Carbonic anhydrase II-positive pancreatic cells are progenitors for both endocrine and exocrine pancreas after birth. Proc Natl Acad Sci U S A.
Jensen, J., Heller, R.S., Funder-Nielsen, T., Pedersen, E.E., Lindsell, C., Weinmaster, G., Madsen, O.D., and Serup, P. (2000). Independent development of pancreatic alpha- and beta-cells from neurogenin3-expressing precursors: a role for the notch pathway in repression of premature differentiation. Diabetes 49, 163-176.
Johansson, K.A., Dursun, U., Jordan, N., Gu, G., Beermann, F., Gradwohl, G., and Grapin-Botton, A. (2007). Temporal control of neurogenin3 activity in pancreas progenitors reveals competence windows for the generation of different endocrine cell types. Dev Cell 12, 457-465.
Jonsson, J., Carlsson, L., Edlund, T., and Edlund, H. (1994). Insulin-promoter-factor 1 is required for pancreas development in mice. Nature 371, 606-609.
Langer (1990), Science 249:1527-1533
Lee, J.C., Smith, S.B., Watada, H., Lin, J., Scheel, D., Wang, J., Mirmira, R.G., and German, M.S. (2001). Regulation of the pancreatic pro-endocrine gene neurogenin3. Diabetes 50, 928-936.
Lieber M, Mazzetta J, Nelson-Rees W, Kaplan M, Todaro G (1975) Establishment of a continuous tumor-cell line (panc-1) from a human carcinoma of the exocrine pancreas. Int J Cancer. 15(5):741-747.
Lois C, Hong EJ, Pease S, Brown EJ, Baltimore D. (2002) Germline transmission and tissue-specific expression of transgenes delivered by lentiviral vectors. Science 295: 868-872, 2002.
Nolan AL, O'Dowd JF (2009). The measurement of insulin secretion from isolated rodent islets of Langerhans. Methods Mol Biol. 560:43-51. Humana Press
Offield, M.F., Jetton, T.L., Labosky, P.A., Ray, M., Stein, R.W., Magnuson, M.A., Hogan, B.L., and Wright, C.V. (1996). PDX-1 is required for pancreatic outgrowth and differentiation of the rostral duodenum. Development 122, 983-995.
Petersson, B., and Hellman, B. (1963). Effects of Long Term Administration of Glucagon on the Pancreatic Islet Tissue of Rats and Guinea-Pigs. Acta Endocrinol (Copenh) 44, 139-149.
Pipeleers, Experientia, 1984.
Powers AC, Efrat S, Mojsov S, Spector D, Habener JF, Hanahan D. (1990) Diabetes. 39(4):406-414. Proglucagon processing similar to normal islets in pancreatic alpha-like cell line derived from transgenic mouse tumor.
Ravassard, P. Chatail, F. Mallet, J. & Icard-Liepkalns, C. (1997) Relax, a novel bHLH transcriptional regulator transiently expressed in the ventricular proliferating zone of the developing central nervous system. J. Neurosc. Res. 48, 146-158.
Rogers GJ et al., Cell Physiol Biochem, 2007.
Rosenberg, L. (1998). Induction of islet cell neogenesis in the adult pancreas: the partial duct obstruction model. Microsc Res Tech 43, 337-346
Salmon P and Trono D (2006) Design and Production of Human Immunodeficiency Virus-Derived Vectors. Cell Biology, a labboratory handbook; third edition, Vol 1, chapter 52, pp 425-434.
Sambrook et al (2001) Molecular cloning Cold Spring Harbor Laboratory Press, Cold Spring Harbor, NY
Seymour, P.A., Freude, K.K., Tran, M.N., Mayes, E.E., Jensen, J., Kist, R., Scherer, G., and Sander, M. (2007). SOX9 is required for maintenance of the pancreatic progenitor cell pool. Proc Natl Acad Sci USA, 1865-1870.
Soriano, P. (1999). Generalized lacZ expression with the ROSA26 Cre reporter strain. Nat Genet 21, 70-71.
Sosa-Pineda, B., Chowdhury, K., Torres, M., Oliver, G., and Gruss, P. (1997). The Pax4 gene is essential for differentiation of insulin-producing beta cells in the mammalian pancreas. Nature 386, 399-402.
Stangé G., Van De Casteele M, and Heimberg H. (2003). Purification of rat pancreatic B-cells by fluorescence-activated cell sorting. Methods Mol Med 83, 15-22.
Theis, M., Mas, C., Doring, B., Degen, J., Brink, C., Caille, D., Charollais, A., Kruger, O., Plum, A., Nepote, V., et al. (2004). Replacement by a lacZ reporter gene assigns mouse connexin36, 45 and 43 to distinct cell types in pancreatic islets. Exp Cell Res 294, 18-29.
Thompson, Nucl. Acids Res (1994) 2:4673-4680
Wang, Q., Elghazi, L., Martin, S., Martins, I., Srinivasan, R.S., Geng, X., Sleeman, M., Collombat, P., Houghton, J., and Sosa-Pineda, B. (2008). Ghrelin is a novel target of Pax4 in endocrine progenitors of the pancreas and duodenum. Dev Dyn 237, 51-61.
Webb, G.C., Akbar, M.S., Zhao, C., Swift, H.H., and Steiner, D.F. (2002). Glucagon replacement via micro-osmotic pump corrects hypoglycemia and alpha-cell hyperplasia in prohormone convertase 2 knockout mice. Diabetes 51, 398-405.
Xu, X., D'Hoker, J., Stange, G., Bonne, S., De Leu, N., Xiao, X., Van De Casteele, M., Mellitzer, G., Ling, Z., Pipeleers, D., et al. (2008). Beta cells can be generated from endogenous progenitors in injured adult mouse pancreas. Cell 132, 197-207.
Zahn, S., Hecksher-Sorensen, J., Pedersen, I.L., Serup, P., and Madsen, O. (2004). Generation of monoclonal antibodies against mouse neurogenin 3: a new immunocytochemical tool to study the pancreatic endocrine progenitor cell. Hybrid Hybridomics, 385-388.
Zhao T, Bokoch GM (2007). Transduction of proteins into intact neutrophils. Methods Mol Biol. 412:115-23
Zhou, Q., Brown, J., Kanarek, A., Rajagopal, J., and Melton, D.A. (2008). In vivo reprogramming of adult pancreatic exocrine cells to β-cells. Nature 455, 627-632.
US 6,071,697
WO 2006/008653

## Claims

1. An *in vitro* method for the generation of pancreatic β-cells, comprising the step of providing at least one pancreatic α-cell or at least one precursor cell with Pax-4 or a nucleic acid encoding Pax-4.

2. The method of claim 1, wherein said method includes providing at least one pancreatic α-cell or at least one precursor cell as the starting material.

3. The method of any of claims 1 or 2, wherein the precursor cell is a pancreatic precursor cell, a small intestine precursor cell, a liver precursor cell or a precursor cell contained within the duct population.

4. Pax-4 or a nucleic acid encoding Pax-4 for use in a method for the generation of pancreatic β-cells *in vivo*, wherein said method includes providing at least one pancreatic α-cell or at least one precursor cell with Pax-4 or a nucleic acid encoding Pax-4 *in vivo.*

5. The method of any of claims 1 to 3 or the protein or the nucleic acid of claim 4, wherein said nucleic acid is a DNA.

6. The method or the protein or the nucleic acid of claim 5, wherein the DNA is comprised in a vector.

7. The method of any of claims 1 to 3 or the protein or the nucleic acid of claim 4, wherein said nucleic acid is an mRNA.

8. The method of any of claims 1 to 3 or 5 to 7 or the protein or the nucleic acid of any of claims 4 to 7, wherein said method does not include providing other proteins involved in cellular development or nucleic acids encoding proteins involved in cellular development.

9. The method of any of claims 1 to 3 or 5 to 7 or the protein or the nucleic acid of any of claims 4 to 7, wherein said method does not include providing other proteins apart from marker proteins or other genes apart from genes encoding marker proteins.

10. The nucleic acid or the protein of any of claims 4 to 9, wherein said method is for the treatment of a pancreatic disease.

11. The nucleic acid or the protein of claim 10, wherein said pancreatic disease is diabetes.

12. A method for the screening of a pancreatic-β-cell phenotype-inducing compound, comprising the step of
a) contacting at least one pancreatic α-cell or at least one precursor cell with a given compound, and
b) testing whether said compound is capable of inducing a pancreatic-β-cell phenotype.

13. The method of claim 12, wherein said testing includes determining whether after the contacting the cell is capable of producing insulin.

14. The method of claim 12, wherein said testing includes determining whether after the contacting the expression of Pax-4 is increased in the cell.

15. The method of any of claims 12 to 14, wherein said method is performed *in vitro.*
